# FASCICULE DE BREVET EUROPEEN

(11) **EP 3 458 860 B1**
(45) Date de publication et mention de la délivrance du brevet: **17.03.2021**
(21) Numéro de dépôt: 17726243.3
(22) Date de dépôt: 22.05.2017
(51) Int. Cl.: G01N 15/14, G01N 15/10, G01N 33/50, G01N 33/574

(54) **DETECTION DE LA MALADIE RESIDUELLE DU MYELOME MULTIPLE**
RESTKRANKHEITSDETEKTION BEI MULTIPLEM MYELOM
RESIDUAL DISEASE DETECTION IN MULTIPLE MYELOMA

(30) Priorité: 20.05.2016 FR 1654514
(43) Date de publication de la demande: 27.03.2019
(73) Titulaire: Horiba ABX SAS, 34184 Montpellier Cedex 4 (FR); Centre Hospitalier Universitaire de Montpellier, 34000 Montpellier (FR); Institut National de la Santé et de la Recherche Médicale (INSERM), 75013 Paris (FR); Université de Montpellier, 34090 Montpellier (FR)
(72) Inventeur: REQUIRAND, Guilhem, 34280 Carnon (FR); KLEIN, Bernard, 34980 Saint Clément de Rivière (FR); REME, Thierry, F-34270 Sainte Croix de Quintillargues (FR); MOREAUX, Jérôme, 34090 Montpellier (FR); RAIMBAULT, Sébastien, 34380 Argelliers (FR); NERIN, Philippe, 34820 Assas (FR); PLANTEFEVE, Rosalie, Montreal, Québec H2R258 (CA); ALATERRE, Elina, 34570 Montarnaud (FR); GARCIA, Jean-Michel, 31330 Larra (FR)
(74) Mandataire: Regimbeau
(86) Numéro de dépôt international: PCT/EP2017/062307
(87) Numéro de publication internationale: WO 2017/198879

(56) Documents cités:
- EP-A1- 2 259 065
- ANOUK CARAUX ET AL: "Residual malignant and normal plasma cells shortly after high dose melphalan and stem cell transplantation. Highlight of a putative therapeutic window in Multiple Myeloma?", ONCOTARGET, vol. 3, no. 11, 25 octobre 2012 (2012-10-25), pages 1335-1347, XP055350322, DOI: 10.18632/oncotarget.650 cité dans la demande
- Lucie Rihova ET AL: "Immunophenotyping in Multiple Myeloma and Others Monoclonal Gammopathies" In: "Multiple Myeloma - A Quick Reflection on the Fast Progress", 10 avril 2013 (2013-04-10), InTech, XP055186603, ISBN: 978-9-53-511083-5 pages 93-110, DOI: 10.5772/55938, le document en entier tableau 2, tube 2 page 93, section 1. (Introduction) page 94, alinéa 3 tableau 1 pages 101-103, section 6. (Clinical application of flow cytometry in MGs)
- N ROBILLARD ET AL: "A single-tube multiparameter seven-colour flow cytometry strategy for the detection of malignant plasma cells in multiple myeloma", BLOOD CANCER JOURNAL, vol. 3, no. 8, 1 août 2013 (2013-08-01), page e134, XP055351211, DOI: 10.1038/bcj.2013.33
- TEMBHARE PRASHANT R ET AL: "Flow cytometric differentiation of abnormal and normal plasma cells in the bone marrow in patients with multiple myeloma and its precursor diseases", LEUKEMIA RESEARCH, NEW YORK,NY, US, vol. 38, no. 3, 11 décembre 2013 (2013-12-11), pages 371-376, XP028665470, ISSN: 0145-2126, DOI: 10.1016/J.LEUKRES.2013.12.007
- MURIELLE ROUSSEL ET AL: "Front-Line Transplantation Program With Lenalidomide, Bortezomib, and Dexamethasone Combination As Induction and Consolidation Followed by Lenalidomide Maintenance in Patients With Multiple Myeloma: A Phase II Study by the Intergroupe Francophone du Myélome", JOURNAL OF CLINICAL ONCOLOGY, vol. 32, no. 25, 1 septembre 2014 (2014-09-01), pages 2712-2717, XP055351210, US ISSN: 0732-183X, DOI: 10.1200/JCO.2013.54.8164
- JUAN FLORES-MONTERO ET AL: "Immunophenotype of normal vs. myeloma plasma cells: Toward antibody panel specifications for MRD detection in multiple myeloma", CYTOMETRY. PART B, CLINICAL CYTOMETRY, vol. 90, no. 1, 31 juillet 2015 (2015-07-31), pages 61-72, XP055351021, US ISSN: 1552-4949, DOI: 10.1002/cyto.b.21265
- KUMAR S ET AL: "Immunophenotyping in multiple myeloma and related plasma cell disorders", BEST PRACTICE & RESEARCH CLINICAL HAEMATOLOGY, ELSEVIER, AMSTERDAM, NL, vol. 23, no. 3, 1 septembre 2010 (2010-09-01), pages 433-451, XP027521905, ISSN: 1521-6926 [extrait le 2010-09-01]
- Anonymous: "BD Horizon(TM) Technical Data Sheet PE-CF594 Mouse Anti-Human Ig, [kappa] Light Chain Product Information", , 1 janvier 2011 (2011-01-01), XP055351216, Extrait de l'Internet: URL:http://www.bdbiosciences.com/ds/pm/tds /562620.pdf [extrait le 2017-03-02]
- Anonymous: "FITC,Mouse,Anti-Human,Light,Chain,[lambda ],JDC-12,RUO - 555796 | BD Biosciences-CA", , 1 janvier 2016 (2016-01-01), XP055351218, Extrait de l'Internet: URL:http://www.bdbiosciences.com/ca/applic ations/research/b-cell-research/immunoglob ulins/human/fitc-mouse-anti-human-light-ch ain-jdc-12/p/555796 [extrait le 2017-03-02]

## Description

La présente invention se situe dans le domaine du diagnostic ou d'aide au diagnostic et du suivi de la maladie résiduelle des hémopathies malignes et a pour objet un procédé de détection de cellules plasmocytaires tumorales et un procédé de diagnostic ou d'aide au diagnostic et de suivi de gammapathies monoclonales tels que définis dans les revendications.

Le myélome multiple (MM) ou maladie de Kahler est une hémopathie maligne caractérisée par l'accumulation de plasmocytes tumoraux (lymphocytes B) dans la moelle osseuse, et l'accumulation d'une immunoglobuline monoclonale complète ou d'une chaîne légère monoclonale. Cette pathologie touche 2 000 nouveaux patients par an en France, 19 000 en Europe, 19 000 aux Etats-Unis. L'espérance de survie médiane des patients d'âge inférieur ou égal à 65 ans est de 6-7 ans. Le diagnostic du MM inclut des signes osseux, une vitesse de sédimentation augmentée, la présence d'une immunoglobuline monoclonale et par un myélogramme montrant :
- la présence de plasmocytes dystrophiques (cytoplasme normal mais anomalies cytologiques nucléaires (cellules multinuclées, mitose multiple ou atypique)) ; et
- une plasmocytose, à savoir soit l'existence d'une plasmocytose supérieure ou égale à 10 % (critère mineur), soit dans certains cas une plasmocytose supérieure à 30 % (critère majeur, pathologie sévère).

Le MM est précédé dans tous les cas d'une gammapathie monoclonale bénigne (MGUS de l'anglais Monoclonal Gammapathy of Undeterminated Significance). Les MGUS sont détectées chez 2 à 4% des sujets de 60 ans, 10% des sujets de 70 ans, 20% des sujets de 80 ans. Les MGUS évoluent vers un MM avec un taux de transformation de 1% par an, indépendant de la date de diagnostic de la MGUS.

Le MM représente 1% des nouveaux cas de cancer par an, 10 à 15% des hémopathies malignes et est la seconde hémopathie maligne la plus fréquente après le lymphome non-Hodgkinien. De plus, les coûts associés à son traitement sont parmi les plus élevés.

Les données récentes de la littérature montrent que l'identification et la caractérisation des cellules plasmocytaires tumorales et normales chez un patient atteint de MM fournissent un marqueur fiable de la réponse au traitement. Le groupe du Pr San Miguel (Paiva et al., 2014) a montré que, lorsqu'il y a moins d'1 cellule plasmocytaire tumorale pour 10 000 cellules leucocytaires totales (avec une sensibilité de 10⁻⁴) trois mois après une chimiothérapie à haute dose suivie d'une autogreffe de cellules souches hématopoïétiques, les patients ont une survie significativement augmentée. Le ratio plasmocytes tumoraux/plasmocytes normaux est un facteur pronostique indépendant des marqueurs pronostiques usuels (Mateo et al., 2008). L'index de prolifération des plasmocytes tumoraux, calculé par cytométrie de flux, est également un puissant facteur pronostique. Il peut être décisif pour évaluer si un patient est en rechute active ou non. Le taux médian de cellules en phase S est de 1% chez un malade (Paiva et al., 2012) et est augmenté chez les patients en rechute.

Par ailleurs, la maladie résiduelle du myélome multiple (MRD - ou en Anglais « Minimal Residual Disease ») désigne la persistance de cellules tumorales résiduelles après traitement. Les plasmocytes représentent jusqu'à 5% des cellules dans la moelle, et la présence de MRD est définie par la présence d'au moins 50 cellules plasmocytaires tumorales pour 500 000 cellules leucocytaires totales, soit 0,01% des cellules (Rawstron et al., 2013).

La durée de survie médiane d'un patient est de 7 mois sans traitement, de 3 à 4 ans avec un traitement par chimiothérapie conventionnelle et de 6 à 7 ans par un traitement avec chimiothérapie à haute dose et autogreffe. Ainsi, le suivi de la MRD après traitement est également très important afin de détecter au plus tôt la rechute et améliorer la prise en charge thérapeutique.

A ce jour, il existe différentes méthodes pour détecter la MRD, par exemple le « Deep Sequencing », la PCR («Polymerase Chain Reaction ») quantitative avec des amorces spécifiques et la cytométrie en flux multiparamétrique (MFC - « Multiparametric Flow Cytometry ») (Martinez-Lopez et al., 2014; Paiva et al., 2008). Les deux premières méthodes ont une sensibilité importante, cependant elles restent très couteuses, longues et sont difficilement automatisables. Ainsi, la cytométrie en flux multicouleurs est à l'heure actuelle la méthode la plus adaptée pour évaluer la réponse des patients, pour détecter précocement la rechute et améliorer la prise en charge thérapeutique afin de maximiser le ratio bénéfice/coût du traitement.

Etant donné la très faible quantité de plasmocytes normaux présents dans la moelle osseuse, soit en valeur médiane 0,44% des cellules mononuclées chez un donneur sain, ceci nécessite d'acquérir un grand nombre d'événements cellulaires pour minimiser les erreurs statistiques.

Actuellement, une méthodologie mise en place par le laboratoire STI (Caraux et al., 2010, 2012) prend 2 jours, soit 16 heures de travail, pour rendre un résultat aux cliniciens. De plus, une telle méthode utilise au moins sept couleurs, sept détecteurs, deux lasers et cinq tubes, donc un nombre important et onéreux de dispositifs et de réactifs. En outre l'utilisation d'une telle méthode nécessite les compétences d'un expert en cytométrie pour la préparation, l'acquisition, la compensation et l'analyse des données.

Il existe donc un réel besoin de développer de nouveaux procédés et compositions pour le diagnostic et le suivi de l'évolution de cette maladie, et notamment d'améliorer la sensibilité et la fiabilité des tests de détection des plasmocytes tumoraux, tout en diminuant les coûts et la complexité des différentes étapes expérimentales.

Les inventeurs ont mis au point une méthode de détection des plasmocytes tumoraux présentant une très grande sensibilité, reproductibilité et fiabilité.

La méthode selon l'invention permet, grâce à l'utilisation d'une combinaison particulière de marqueurs de cellules plasmocytaires tumorales et normales, d'obtenir des résultats de détection des plasmocytes normaux et tumoraux au moins équivalents à ceux obtenus à partir des méthodes de détection déjà connues dans l'état de l'art.

Par rapport aux méthodes de l'état de l'art, le procédé selon l'invention présente l'avantage de pouvoir être réalisé dans un contenant unique, tel qu'un tube ou un puits d'une plaque à puits, comprenant l'échantillon à analyser et les différents marqueurs. Ceci présente en particulier l'avantage d'être moins coûteux, de diminuer la durée et la complexité des différentes étapes expérimentales, et de réduire le délai d'obtention des résultats. Ainsi, la méthode selon l'invention permet de diminuer les besoins en personnel qualifié et les risques d'erreurs associés, augmentant ainsi dans le même temps la fiabilité de la méthode. De plus, la diminution de la complexité des différentes étapes expérimentales du procédé selon l'invention permet d'améliorer la reproductibilité et d'augmenter la productivité.

L'invention a pour objet un procédé de détection par cytométrie en flux de la présence de cellules plasmocytaires normales et de cellules plasmocytaires tumorales dans un échantillon de cellules d'un patient, ledit procédé comprenant les étapes suivantes:
a) la mise en contact dudit échantillon de cellules avec :
   - un marqueur de la chaîne κ, un marqueur de la chaîne λ, et un marqueur choisi parmi :
      un marqueur de CD38 et un marqueur de CD138, chacun des trois marqueurs émettant un signal différent (ci-après les 'trois premiers signaux'), et
   - un ou plusieurs marqueur(s) de cellules plasmocytaires tumorales, chacun de ces un ou plusieurs marqueur(s) émettant un seul et même signal autrement nommé 'signal détectable unique' (ci-après le 'quatrième' signal), différent des trois premiers signaux, et
   - un ou plusieurs marqueur(s) de cellules plasmocytaires normales, chacun de ces un ou plusieurs marqueur(s) émettant un seul et même signal autrement nommé 'signal détectable unique' (ci-après le 'cinquième signal'), différent des quatre premiers signaux,
b) la détection des cellules plasmocytaires caractérisées par :
   - un signal positif, en particulier un signal positif fort, avec un marqueur de CD38 ou un marqueur de CD138, et
   - un signal positif, en particulier un signal positif fort, avec un marqueur de la chaîne κ ou un marqueur de la chaîne λ,
c) la détection des cellules plasmocytaires tumorales parmi les cellules plasmocytaires détectées à l'étape b) présentant au moins un des critères suivants (les autres plasmocytes étant considérés comme normaux) :
   - signal positif avec au moins un marqueur spécifique de cellules plasmocytaires tumorales, et/ou
   - un signal négatif ou significativement réduit avec au moins un marqueur de cellules plasmocytaires normales,
d) l'analyse des résultats obtenus lors des étapes b) et c) et la quantification des cellules plasmocytaires normales et des cellules plasmocytaires tumorales.

Selon d'autres modes de réalisation, on pourra utiliser comme alternative aux marqueurs respectifs de CD38 et de CD138, un ou plusieurs marqueurs choisis dans le groupe constitué de : un marqueur de CD229, un marqueur de CD54, un marqueur de CD319 (Pojero et al., Utility of CD54, CD229, and CD319 for the identification of Plasma Cells in Patients with Clonal Plasma Cell Diseases), un marqueur de CD319, ou un marqueur de CD269 (Frigyesi et al., Blood. 2014 Feb 27;123(9):1336-40. doi: 10.1182/blood-2013-09-529800). On peut également citer un marqueur de MUM1/IRF4 qui est un marqueur intracytoplasmique.

Les marqueurs précités peuvent être utilisés individuellement, en remplacement des marqueurs respectifs de CD38 et de CD138, ou en combinaison entre eux et/ou notamment avec CD38.

On pourra ainsi utiliser par exemple un marqueur de la chaîne κ, un marqueur de la chaîne λ, et un marqueur de CD229.

On comprend du reste de la description et des exemples illustratifs de l'invention décrits ci-après que le ou les marqueurs CD selon l'invention, pour un même groupe de marqueurs ayant un différentiel d'expression commun (diminution ou perte *versus* augmentation ou gain), sont couplés ou conjugués à un même fluorochrome, ou chromophore.

Selon la présente invention, d'une part le ou les marqueur(s) de cellules plasmocytaires tumorales à l'étape a) émettent un (quatrième) signal différent des trois premiers signaux, au sens de un seul et même signal différent des trois premiers signaux, autrement dit un signal commun ou un 'signal détectable unique' différent des trois premiers signaux.

Par 'un seul et même signal' ou 'signal détectable unique', on entend selon l'invention que le ou les marqueurs des cellules plasmocytaires tumorales sont caractérisés en ce qu'ils émettent un signal commun, reconnu par le détecteur.

Selon un mode de réalisation préféré, le ou lesdits marqueurs de cellules plasmocytaires tumorales sont couplés ou conjugués au même fluorochrome pour émettre un seul et même signal autrement nommé 'signal détectable unique', différent des trois premiers signaux.

Selon une alternative, le ou lesdits marqueurs de cellules plasmocytaires tumorales sont couplés ou conjugués à des fluorochromes qui émettent des signaux détectables dans des bandes de fréquences proches, équivalents ou assimilables à un seul et même signal autrement nommé 'signal détectable unique' par un détecteur de fréquence adaptée, ledit signal détectable unique étant différent des trois premiers signaux.

Selon la présente invention, d'autre part le ou les marqueur(s) de cellules plasmocytaires normales à l'étape a) émettent un (cinquième) signal différent des quatre premiers signaux, au sens de un seul et même signal différent des quatre premiers signaux, autrement dit un signal commun ou un 'signal détectable unique' différent des quatre premiers signaux.

Par 'un seul et même signal' ou 'signal détectable unique', on entend selon l'invention que le ou les marqueurs des cellules plasmocytaires normales sont caractérisés en ce qu'ils émettent un signal commun, reconnu par le détecteur.

Selon un mode de réalisation préféré, le ou lesdits marqueurs de cellules plasmocytaires normales sont couplés ou conjugués au même fluorochrome pour émettre un seul et même signal autrement nommé 'signal détectable unique', différent des quatre premiers signaux.

Selon une alternative, le ou lesdits marqueurs de cellules plasmocytaires normales sont couplés ou conjugués à des fluorochromes qui émettent des signaux détectables dans des bandes de fréquences proches, équivalents ou assimilables à un seul et même signal autrement nommé 'signal détectable unique' par un détecteur de fréquence adaptée, ledit signal détectable unique étant différent des quatre premiers signaux.

La détection d'un seul et même signal pour un même groupe de marqueurs ayant un différentiel d'expression commun (diminution ou perte *versus* augmentation ou gain), permet avantageusement de diminuer le nombre de fluorochromes et/ou de signaux détectables, donc le nombre de détecteurs et de sources dans le procédé de détection selon l'invention, pour une analyse rapide (fichiers de données plus petits) et simplifiée.

Préférentiellement, le marqueur de cellules plasmocytaires selon l'invention est un marqueur d'antigène de cellules plasmocytaires, préférentiellement un marqueur de cluster de différenciation (CD) de cellules plasmocytaires.

Par « marqueur » on entend toute(s) molécule(s) permettant la reconnaissance de la cible et sa détection par l'émission d'un signal détectable. Avantageusement, pour un marquage de séquence peptidique, les marqueurs selon l'invention sont des aptamers ou des anticorps conjugués à des fluorochromes, préférentiellement les fluorochromes sont sélectionnés parmi PE-Cy5.5, PE-CF594, PE-Cy7, PE, APC et FITC. Alternativement, pour un marquage de l'ADN, les marqueurs selon l'invention peuvent également être des analogues de base, des molécules autofluorescentes lorsqu'elles se lient ou s'intercalent à l'ADN (par exemple le DAPI ; fluorescence bleue à environ 456nm) ou des molécules conjuguées à des fluorochromes (par exemple des anticorps) pouvant se lier ou s'intercaler à l'ADN. Avantageusement selon l'invention, le ou les marqueurs des cellules plasmocytaires tumorales sont conjugués à un même fluorochrome (chromophore) pour l'émission d'un seul et même signal, distinct des autres signaux. Et le ou les marqueurs des cellules plasmocytaires normales sont conjugués à un autre fluorochrome (chromophore) pour l'émission d'un seul et même signal, distinct des autres signaux.

Cela permet d'obtenir un seul et même signal par groupe de marqueurs ayant un différentiel d'expression commun (diminution ou perte *versus* augmentation ou gain), simplifiant le procédé et permettant avantageusement une mise en œuvre dans un contenant unique.

Ainsi, la quantité de signal émis par un marqueur selon l'invention est liée à la quantité de complexes marqueur/cible, et donc au niveau d'expression de séquences peptidiques et/ou à la quantité d'ADN cible.

Par « signal», on entend tout signal émis avec des propriétés qui permettent de recouvrer ce signal après transmission. Ces propriétés peuvent par exemple être des fréquences, des modulations, des encodages. Préférentiellement, les signaux détectables sont émis dans des bandes de fréquences qui leur sont allouées, plus préférentiellement des couleurs du spectre visible dans une transmission optique. Aussi, différents marqueurs qui émettent différents signaux détectables dans des bandes de fréquences proches peuvent être détectés par un même détecteur, ou différents marqueurs qui émettent un même signal détectable unique peuvent être détectés par un même détecteur.

Selon un mode particulier et préféré, on utilisera selon l'invention un ou plusieurs marqueurs émettant un seul et même signal autrement nommé 'signal détectable unique' pour un même groupe de marqueurs ayant un différentiel d'expression commun (diminution ou perte *versus* augmentation ou gain), distinct des autres signaux.

A chaque groupe de marqueurs ayant un différentiel d'expression commun (diminution ou perte *versus* augmentation ou gain), correspond ainsi un même fluorochrome (chromophore), pour l'émission d'un seul signal.

Par « détecteur » on entend tout système/dispositif permettant de recouvrer un des signaux détectables parmi les autres, sa détection et/ou sa quantification. Préférentiellement, le détecteur utilise un filtre pour isoler le signal qu'il détecte. Plus préférentiellement, ce filtre est de type passe-bande. Préférentiellement, la détection et/ou la quantification est réalisée par un convertisseur photonique/électronique, par exemple des photodiodes, photodiodes à avalanche, photomultiplicateurs (PMT).

Par « plasmocyte normal » on entend des plasmocytes, cellules plasmatiques ou plasmatocytes présentant les antigènes (Cluster de différenciation ; CD) sélectionnés parmi, CD19, CD27, CD38, CD45, CD81, CD5L, CD11a, CD16b, CD24, CD36, CD52, CD68, CD79a, CD80, CD82, CD84, CD99, CD138, CD163, et ne présentant pas les antigènes CD20, CD28, CD52, CD56, CD117 et CD200. Les plasmocytes normaux présentent les chaînes intra cytoplasmiques kappa ou lambda avec des proportions respectives de 2/3 et 1/3.

Par « plasmocyte tumoral » on entend des plasmocytes, cellules plasmatiques ou plasmatocytes présentant au moins une altération par rapport au profil normal :
- perte d'au moins un CD parmi CD19, CD27, CD45, CD52 et CD81,
- gain d'au moins un CD parmi CD20, CD28, CD56, CD117, CD200 et CD229,
- changement du niveau d'expression d'au moins un CD parmi CD5L, CD11a, CD16b, CD24, CD36, CD52, CD68, CD79a, CD80, CD82, CD84, CD99, CD138 et CD163, sous-exprimés dans les plasmocytes tumoraux, CD1D, CD2BP2, CD32c, CD47, CD59, CD109, CD138, CD300a et CD320, surexprimés dans les plasmocytes tumoraux et/ou
- altération du ratio kappa/lambda (monoclonalité).

L'expression « signal positif » signifie selon l'invention que le signal est dit positif lorsque les marqueurs spécifiques de cellules plasmocytaires utilisés se lient à leurs cibles et émettent un signal significativement détectable. Le « signal positif » montre ainsi que la ou les cellule(s) plasmocytaire(s) détectée(s) à l'étape b) du procédé selon l'invention présente(nt) un niveau d'expression significativement augmenté de la molécule reconnue par le(s) marqueur(s) utilisé(s) par la ou les cellule(s) plasmocytaire(s) détectée(s). Un signal positif pour un marqueur 'CD' se traduit communément par 'CD⁺' ou par les terminologies 'CD^{faible}', 'CD^{moyen}/CD^{intermediaire}' ou 'CD^{fort}' (autrement nommés en anglais CD^{low or dim}, CD^{mid or intermediaite} et CD^{high or bright}) en fonction du niveau d'expression dudit marqueur détecté. On parlera également de signal positif faible, moyen ou fort.

L'intensité de fluorescence observée sur des matrices telles que celles illustrées dans les exemples ci-après, avec en abscisse l'intensité de fluorescence mesurée, est usuellement classifiée selon les ordres de grandeurs suivants (décades), à titre illustratif :
Inférieure à 10² : signal négatif ('negative' en anglais), ou '-' ;
De 10² à 10³ : signal positif faible ('low' or 'dim' en anglais), ou '+/-' ;
De 10³ à 10⁴ : signal positif moyen ('mid' or 'intermediate'), ou '+';
Supérieure à 10⁴ : signal positif fort ('high' or 'bright' en anglais), ou '++'.

Selon l'invention, et comme illustré dans les exemples et figures ci-après, on s'intéresse en particulier à la détection d'un signal positif fort pour la détection des cellules plasmocytaires tumorales à l'étape b) du procédé de détection selon l'invention. Cette étape b) de détection des cellules plasmocytaires (totaux) est caractérisée par : un signal positif fort avec un marqueur de CD38 ou un marqueur de CD138, et un signal positif fort avec un marqueur de la chaîne κ ou un marqueur de la chaîne λ. Cela peut également se traduire par les terminologies CD38⁺⁺/κ⁺⁺ ou CD138⁺⁺/λ⁺⁺. Par exemple ou CD38^{fort}/κ^{fort} ou CD138^{fort}/λ^{fort}. Cette étape b) de détection d'un signal positif fort des cellules plasmocytaires (totaux) sur-exprimant un marqueur CD38 ou CD138 et un marqueur de la chaîne κ ou un marqueur de la chaîne λ est avantageuse en ce qu'elle permet de s'affranchir d'étapes additionnelles visant à éliminer d'autres types cellulaires telles que des lymphocytes B. Le procédé de détection selon l'invention est donc simplifié par rapport aux procédés de détection actuels.

Par « signal négatif » on entend que le signal est dit négatif lorsque les marqueurs spécifiques de cellules plasmocytaires utilisés ne se lient pas, ou se lient faiblement, à leurs cibles et n'émettent donc pas de signal significativement détectable. Le « signal négatif » de ces marqueurs spécifiques montre ainsi que la ou les cellule(s) plasmocytaire(s) détectée(s) à l'étape b) du procédé selon l'invention présente un niveau d'expression négatif ou significativement réduit de la molécule reconnue par le(s) marqueur(s) utilisé(s). Un signal négatif pour un marqueur 'CD' se traduit communément par 'CD'.

Préférentiellement, l'échantillon de cellules utilisé pour le procédé selon l'invention est choisi parmi, ou obtenu à partir de la moelle osseuse, du sang, du sérum, d'un extrait sanguin, des PBMCs (*Peripheral Blood Mononuclear Cells*)*,* du liquide céphalorachidien, du liquide pleural et des ganglions lymphatiques, préférentiellement de la moelle osseuse d'un patient.

Selon un mode de réalisation particulier, l'échantillon de cellules du procédé selon l'invention provient d'un patient atteint de gammapathie monoclonale, ou d'une personne susceptible d'être atteinte d'une gammapathie monoclonale.

Selon un mode de réalisation préféré, les CD spécifiques de cellules plasmocytaires normales du procédé selon l'invention sont choisis parmi : CD19, CD27, CD45, CD81, CD5L, CD11a, CD16b, CD24, CD36, CD52, CD68, CD79a, CD80, CD82, CD84, CD99 et CD163, préférentiellement CD19, CD27, CD45 et CD81 plus préférentiellement CD19, CD27.

Selon un mode de réalisation préféré, les CD spécifiques de cellules plasmocytaires tumorales du procédé selon l'invention sont choisis parmi : CD56, CD117, CD200, CD20, CD28, CD1D, CD2BP2, CD32c, CD47, CD59, CD109, CD229, CD300a et CD320, préférentiellement CD56, CD117, CD200, CD20, et CD28, plus préférentiellement CD56, CD117, CD200.

Préférentiellement, le procédé selon l'invention comprend en outre l'analyse des signaux du FSC (« Forward Scatter ») et du SSC (« Side Scatter »). FSC et SSC correspondent à des mesures simultanées réalisées par cytométrie de flux permettant de déterminer les caractéristiques physiques et biologiques de cellules isolées. Par définition, le FSC correspond à l'intensité de la lumière diffractée de la source d'excitation dans l'axe (angle <12°), qui est proportionnelle à la taille de la cellule, tandis que le SSC correspond à l'intensité de la lumière diffractée orthogonalement, dit « aux grands angles » (proche de 90°), par rapport à la source lumineuse incidente qui est représentative de la complexité intracellulaire.

Selon un mode de réalisation préféré, l'échantillon du procédé selon l'invention est mis en contact avec :
- un marqueur de la chaîne κ, un marqueur de la chaîne λ et un marqueur de CD38 ou CD138, préférentiellement CD38, chacun des trois marqueurs émettant un signal différent, et
- un ou plusieurs marqueur(s), préférentiellement au moins deux marqueurs de CD de cellules plasmocytaires normales sélectionné(s) parmi les marqueurs de CD19, CD27, CD45, CD81, CD5L, CD11a, CD16b, CD24, CD36, CD52, CD68, CD79a, CD80, CD82, CD84, CD99 et CD163, préférentiellement les marqueurs de CD19, CD27, CD45 et CD81, plus préférentiellement un marqueur de CD19 et un marqueur de CD27, chacun de ces un ou plusieurs marqueur(s) émettant un seul et même signal autrement nommé 'signal détectable unique', différent des trois premiers signaux, et
- un ou plusieurs marqueur(s), préférentiellement au moins deux marqueurs, plus préférentiellement au moins trois marqueurs de CD de cellules plasmocytaires tumorales sélectionné(s) parmi les marqueurs de CD56, CD117, CD200, CD20, CD28, CD1D, CD2BP2, CD32c, CD47, CD59, CD109, CD229, CD300a et CD320, préférentiellement les marqueurs de CD56, CD117, CD200, CD20, et CD28, plus **préférentiellement** un marqueur de CD56, un marqueur de CD117 et un marqueur de CD200, chacun de ces un ou plusieurs marqueur(s) émettant un seul et même signal autrement nommé 'signal détectable unique', différent des quatre premiers signaux.

Selon un mode particulier de l'invention, l'échantillon du procédé selon l'invention est mis en contact avec :
(i) un nombre de marqueurs inférieur à dix marqueurs, notamment inférieur ou égal à neuf marqueurs voire inférieur ou égal à huit marqueurs, voire inférieur ou égal à sept marqueurs, voire inférieur ou égal à six marqueurs avec :
   - au moins trois marqueurs des plasmocytes totaux avec un marqueur de la chaîne κ, un marqueur de la chaîne λ et un marqueur de CD38 ou CD138, et
   - au moins un marqueur des cellules plasmocytaires normales, et
   - au moins un marqueur des cellules plasmocytaires tumorales, et
   - optionnellement au moins un marqueur de prolifération cellulaire, et
(ii) un nombre inférieur à huit fluorochromes, notamment inférieur ou égal à sept fluorochromes, voire inférieur ou égal à six fluorochromes voire égal à cinq fluorochromes, avec :
   - trois fluorochromes pour les marqueurs de plasmocytes totaux, et
   - un fluorochrome distinct des précédents pour le ou les marqueurs des cellules plasmocytaires normales, et
   - un fluorochrome pour le ou les marqueurs des cellules plasmocytaires tumorales, et
   - optionnellement un fluorochrome pour le ou les marqueurs de prolifération cellulaire.

Selon un mode particulier et préféré de l'invention, le procédé selon l'invention est réalisé dans un contenant unique, tel qu'un tube ou un puits d'une plaque à puits, comprenant l'échantillon à analyser et les différents marqueurs. De préférence, le procédé selon l'invention est réalisé dans un tube.

Préférentiellement, l'échantillon du procédé selon l'invention est mis en contact avec :
- un marqueur de la chaîne κ, un marqueur de la chaîne λ et un marqueur de CD38, chacun des trois marqueurs émettant un signal différent, et
- un marqueur de CD19 et un marqueur de CD27, émettant un seul et même signal autrement nommé 'signal détectable unique', différent desdits trois premiers signaux, et
- un marqueur de CD56, un marqueur de CD117 et un marqueur de CD200, émettant un seul et même signal autrement nommé 'signal détectable unique', différent des quatre premiers signaux.

Préférentiellement, le marqueur CD38 ou CD138 est respectivement un anticorps anti-CD38 ou anti-CD138 conjugué au fluorochrome PE-Cy5.5, le marqueur de la chaîne κ est un anticorps anti-chaîne κ conjugué au fluorochrome PE-CF594 et le marqueur de la chaîne λ est un anticorps anti-chaîne λ conjugué au fluorochrome FITC ; la détection se faisant sur les détecteurs du cytomètre en flux appropriés selon l'état de l'art.

Avantageusement, les marqueurs CD19 et CD27 sont des anticorps anti-CD19 et anti-CD27 conjugués au fluorochrome PE-Cy7, et les marqueurs CD56, CD117 et CD200 sont des anticorps anti-CD56, anti-CD117 et anti-CD200 conjugués au fluorochrome PE ; la détection se faisant sur les détecteurs du cytomètre en flux appropriés selon l'état de l'art.

De plus, comme indiqué précédemment, l'index de prolifération des plasmocytes tumoraux correspond au pourcentage de cellules tumorales en phase S, calculé en cytométrie de flux, est un puissant facteur pronostique, permettant d'évaluer la vitesse de développement de la masse tumorale et de déterminer si un patient est en rechute active ou non.

Ainsi, selon un mode de réalisation préféré, le procédé selon l'invention comprend en outre la détermination de l'index de prolifération des plasmocytes tumoraux comprenant les étapes suivantes:
A. mise en contact dudit échantillon de cellules avec un ou plusieurs marqueur(s), préférentiellement deux marqueurs, de prolifération cellulaire, émettant un signal différent des autres marqueurs utilisés;
B. détection des plasmocytes tumoraux en prolifération par analyse du signal émis par ledit ou lesdits marqueur(s) ;
C. détermination du pourcentage de cellules tumorales et de plasmocytes normaux étant en phase S ;
D. optionnellement détermination de la diploïdie (G0/G1 ou G2/M) lorsque au moins un du ou desdits marqueur(s) de prolifération cellulaire est un marqueur de l'ADN.

On entend par « marqueurs de prolifération» tous marqueurs permettant de déterminer si une cellule est en phase du cycle cellulaire G0/G1, G2/M ou S. Les marqueurs de prolifération sont par exemple choisis parmi :
- des marqueurs de prolifération permettant d'identifier les cellules en phase S, tel que les analogues de bases de type BrdU ou EdU,
- des marqueurs de prolifération permettant d'identifier les cellules en phases G0 (cellules quiescentes) ou G1/S/G2/M (cellules non quiescentes), par exemple des marqueurs de protéines tel que Ki67 (Dako)
- des marqueurs de prolifération permettant d'identifier les cellules en phases G0/G1 ou G2/M, par exemple des marqueurs d'acides désoxyribonucléiques (ADN), tel que l'acridine orange, DAPI, Iodure de Propidium (PI), colorant Hoeschst (33342 ou 33258);

Selon un mode particulier et préféré, on utilisera au moins un marqueur de prolifération permettant d'identifier les cellules en phase S, tel que les analogues de bases de type BrdU ou EdU, et de préférence de type BrdU.

Selon un mode de réalisation particulier, les marqueurs de la prolifération des plasmocytes tumoraux du procédé selon l'invention sont sélectionnés parmi :
- des analogues de bases de type BrdU reconnu par un anticorps anti-BrdU conjugué à l'allophycocyanine (APC) ou Ethynyldésoxyuridine (EdU) reconnu par un azoture couplé à un fluorochrome, préférentiellement l'Alexa Fluor® 647 ; et/ou
- des protéines qui sont présentes en cycle cellulaire actif, par exemple Ki67 reconnu par un anticorps anti-Ki67 conjugué à un fluorochrome, préférentiellement l'Alexa Fluor 647 ; et/ou
- des marqueurs d'acides désoxyribonucléiques (ADN), tel que le DAPI, PI, colorant Hoeschst (33342 ou 333258), etc...

Le terme « BrdU » (BrdU FlowKit ; BD Pharmingen) est défini comme un nucléoside synthétique, analogue structurel de la thymidine, qui peut être incorporé dans l'ADN et qui peut être détecté par un anticorps spécifique pour déterminer la phase du cycle cellulaire d'une cellule et notamment si cette dernière est en phase de réplication de l'ADN (phase S). Le terme « EdU » (Click-iT® EdU ; Thermofisher) est défini comme un nucléoside synthétique (analogue structurel de la thymidine) qui est incorporé à l'ADN lors de sa réplication. La liaison entre un alcyne du fragment éthynyle de l'EdU et un azoture couplé à un flurochrome est réalisée par réaction de cycloaddition, une des réactions de ligation les plus populaires de la « click-chemistry ». L'EdU peut alors être détectée lors de son incorporation à l'ADN afin de déterminer si les cellules d'intérêt sont en phase de réplication de l'ADN (phase S).

Avantageusement, le procédé de détermination de l'index de prolifération des plasmocytes tumoraux selon l'invention comprend les étapes suivantes:
A1. mise en contact du BromodesoxyUrydine (BrdU) avec un échantillon de cellules (suivant les recommandations du fournisseur ; BrdU FlowKit ; BD Pharmingen ; Cat. : 557892) ;
B1. dénaturation de l'ADN par l'action d'une désoxyribonucléase (DNase, BrdU FlowKit ; BD Pharmingen ; Cat. : 557892) ;
C1. marquage du BrdU incorporé à l'ADN par un anticorps anti-BrdU conjugué à un fluorochrome émettant un signal différent du signal des autres marqueurs utilisés, préférentiellement ledit fluorochrome est l'APC ;
D1. détection en cytométrie en flux desdites cellules marquées par ledit BrdU;
E1. détermination du pourcentage de cellules tumorales et de plasmocytes normaux en phase S.

Préférentiellement, le procédé de détermination de l'index de prolifération des plasmocytes tumoraux selon l'invention comprend les étapes suivantes:
A2. mise en contact d'Ethynyldésoxyuridine (EdU) avec un échantillon de cellules (suivant les recommandations du fournisseur ; Click-it® Plus EdU Alexa Fluor® 647 Flow Cytometry Assay Kit, Life Technologies, Cat. : C10635) ;
B2. marquage de l'EdU avec un fluorochrome émettant un signal différent du signal des autres marqueurs utilisés, préférentiellement ledit fluorochrome est l'Alexa Fluor® 647;
C2. détection en cytométrie en flux desdites cellules marquées par ledit EdU;
D2. détermination du pourcentage de cellules tumorales et de plasmocytes normaux en phase S.

Préférentiellement, le procédé de détermination de l'index de prolifération des plasmocytes tumoraux selon l'invention comprend en outre une étape additionnelle d'évaluation de la diploïdie consistant à ajouter entre les étapes C1 et D1 ou B2 et C2 du DAPI.

Préférentiellement, le procédé de détermination de l'index de prolifération des plasmocytes tumoraux selon l'invention comprend les étapes suivantes:
A3. mise en contact du BromodesoxyUrydine (BrdU) avec un échantillon de cellules (suivant les recommandations du fournisseur ; BrdU FlowKit ; BD Pharmingen ; Cat. : 557892) ;
B3. dénaturation de l'ADN par l'action d'une désoxyribonucléase (DNase, BrdU FlowKit ; BD Pharmingen ; Cat. : 557892) ;
C3. marquage du BrdU incorporé à l'ADN par la fixation d'un anticorps anti-BrdU conjugué à un fluorochrome émettant un signal différent des signaux des autres marqueurs utilisés, préférentiellement ledit fluorochrome est l'APC ;
D3. mise en contact du DAPI avec l'échantillon de cellules ;
E3. détection en cytométrie en flux desdites cellules marquées par lesdits BrdU et DAPI;
F3. détermination de la diploïdie et du pourcentage de cellules tumorales et de plasmocytes normaux étant en phase S.

Préférentiellement, le procédé de détermination de l'index de prolifération des plasmocytes tumoraux selon l'invention comprend les étapes suivantes:
A4. mise en contact d'Ethynyldésoxyuridine (EdU) avec un échantillon de cellules (suivant les recommandations du fournisseur ; Click-it® Plus EdU Alexa Fluor® 647 Flow Cytometry Assay Kit, Life Technologies, Cat. : C10635) ;
B4. marquage de l'EdU avec un fluorochrome émettant un signal différent du signal des signaux des autres marqueurs utilisés, préférentiellement ledit fluorochrome est l'Alexa Fluor® 647 ;
C4. mise en contact du DAPI avec l'échantillon de cellules ;
D4. détection en cytométrie en flux desdites cellules marquées par lesdits EdU et DAPI;
E4. détermination de la diploïdie des cellules tumorales et des plasmocytes normaux.

La proportion de plasmocytes tumoraux par rapport aux autres leucocytes donne une image de l'environnement cellulaire, mais cette proportion est influencée par les variations des autres populations leucocytaires.

La quantification des cellules tumorales donne quant à elle une évaluation de la masse tumorale. Cette quantification peut être réalisée par l'analyse d'un volume connu en cytométrie de flux, par l'utilisation de particules de comptage, tel que des billes de polystyrène fluorescentes (par exemple commercialisées par Coulter, FlowCyto Standard Corporation, Polysciences, Sperotech, BD TrueCounts, DakoCytomation...), introduites en quantité connue dans un volume d'échantillon connu, ou encore indirectement par la proportion de plasmocytes tumoraux par rapport à une population quantifiée par ailleurs (par exemple le nombre de leucocytes totaux de l'échantillon, issu d'un automate de numération).

Ainsi, selon un mode de réalisation particulier, le procédé de détection par cytométrie en flux de la présence de cellules plasmocytaires normales et de cellules plasmocytaires tumorales dans un échantillon de cellules d'un patient selon l'invention comprend en outre :
- une étape de détermination ou de réception de la quantité d'une population ou un groupe de populations cellulaires par unité de volume ;
- la détermination de la proportion des populations plasmocytaires par rapport à la population ou groupe de populations quantifiée(s) ;
- quantification des populations plasmocytaires par unité de volume ;

Par « réception de la quantité d'une population » on entend une action permettant de renseigner le système sur la quantité de cellules présentes dans l'échantillon à analyser ou déjà analysé (ex : quantification des leucocytes). Cette action peut par exemple consister en une transmission informatique, ou une saisie de l'opérateur.

Selon un mode de réalisation particulier, ledit patient est considéré à risque de développer une gammapathie monoclonale lorsque le nombre de cellules plasmocytaires tumorales par unité de volume est supérieur à une valeur seuil prédéfinie. Cette « valeur seuil prédéfinie » est déterminée à partir de résultats obtenus par le procédé selon l'invention à partir de sujets contrôle sains et/ou malades. Selon un mode particulier de réalisation de la présente invention, les valeurs seuils prédéfinies selon l'invention sont paramétrables.

La présente invention a également pour objet un procédé de diagnostic ou d'aide au diagnostic *in vitro* ou de suivi de gammapathie monoclonale, à partir d'un échantillon de cellules d'un patient comprenant les étapes suivantes :
1) mise en œuvre du procédé selon l'invention, et
2) mise en évidence d'un risque de développer une gammapathie monoclonale :
   a) si la proportion de cellules plasmocytaires tumorales parmi les cellules leucocytaires totales, d'après les résultats de l'analyse de l'étape d) du procédé selon l'invention, dépasse un seuil prédéfini; et/ou
   b) si le nombre de plasmocytes tumoraux par unité de volume dépasse un seuil prédéfini.

La présente invention a également pour objet un procédé de diagnostic ou d'aide au diagnostic *in vitro* de gammapathie monoclonale à partir d'un échantillon de cellules d'un patient dans lequel les étapes 1) et 2) selon l'invention sont automatisées.

La présente invention a également pour objet un procédé de suivi *in vitro* de l'évolution de la maladie d'un patient atteint d'une gammapathie monoclonale à partir d'un premier et d'un second échantillon de cellules du patient, comprenant les étapes suivantes :
i) la mise en œuvre du procédé de détection selon l'invention dans un premier échantillon de cellules du patient,
ii) la mise en œuvre du procédé de détection selon l'invention dans un second échantillon de cellules du patient, ledit second échantillon ayant été prélevé après le prélèvement dudit premier échantillon, et
iii) la comparaison de l'analyse des résultats de l'étape d) obtenus avec le premier et le second échantillon, et
iv) le suivi de l'évolution de ladite gammapathie monoclonale (masse tumorale, prolifération, monoclonalité...) dudit patient d'après les résultats de l'analyse de l'étape iii).

La présente invention a également pour objet un procédé de suivi *in vitro* de l'évolution de la maladie d'un patient atteint d'une gammapathie monoclonale, à partir d'un premier et d'un second échantillon de cellules du patient, caractérisé en ce que les étapes i), ii), iii) et iv) sont automatisées.

La présente invention concerne également une composition comprenant au moins :
- un marqueur de la chaîne κ et un marqueur de la chaîne λ, émettant un signal différent, et
- un marqueur choisi parmi : un marqueur de CD38 ou un marqueur de CD138, ledit marqueur émettant un signal différent des deux premiers signaux, et
- un ou plusieurs marqueur(s), préférentiellement au moins deux marqueurs, de CD spécifiques de cellules plasmocytaires normales sélectionnés parmi les marqueurs de CD19, CD27, CD45, CD81, CD5L, CD11a, CD16b, CD24, CD36, CD52, CD68, CD79a, CD80, CD82, CD84, CD99 et CD163, préférentiellement les marqueurs de CD19, CD27, CD45 et CD81, plus préférentiellement un marqueur de CD19 et un marqueur de CD27, chacun de ces un ou plusieurs marqueur(s) émettant un seul et même signal autrement nommé 'signal détectable unique', différent des trois premiers signaux, et
- un ou plusieurs marqueur(s), préférentiellement au moins deux marqueurs, plus préférentiellement au moins trois marqueurs de CD spécifiques de cellules plasmocytaires tumorales sélectionnés parmi les marqueurs de CD56, CD117, CD200, CD20, CD28, CD1D, CD2BP2, CD32c, CD47, CD59, CD109, CD229, CD300a et CD320, préférentiellement les marqueurs de CD56, CD117, CD200, CD20, et CD28, plus préférentiellement un marqueur de CD56, un marqueur de CD117 et un marqueur de CD200, chacun de ces un ou plusieurs marqueur(s) émettant un seul et même signal autrement nommé 'signal détectable unique', différent des quatre premiers signaux.

Avantageusement, la composition selon l'invention comprend au moins :
- un marqueur de la chaîne κ et un marqueur de la chaîne λ, émettant un signal différent ; et
- un marqueur choisi parmi : un marqueur de CD38 et un marqueur de CD138, ledit marqueur émettant un signal différent des deux premiers signaux ; et
- un marqueur de CD19 et un marqueur de CD27, lesdits marqueurs émettant un seul et même signal autrement nommé 'signal détectable unique', différent des trois premiers signaux ; et
- un marqueur de CD56, un marqueur de CD117, un marqueur de CD200, lesdits marqueurs émettant un seul et même signal autrement nommé 'signal détectable unique', différent des quatre premiers signaux.

Selon un mode de réalisation avantageux, la composition selon l'invention comprend en outre un ou plusieurs marqueur(s) de la prolifération de plasmocytes tumoraux selon l'invention, émettant un signal différent de chacun des signaux des autres marqueurs présents dans ladite composition.

La présente invention a également pour objet l'utilisation d'une composition comprenant au moins :
- un marqueur de la chaîne κ et un marqueur de la chaîne λ émettant un signal différent, et
- un marqueur choisi parmi : un marqueur de CD38 et un marqueur de CD138, ledit marqueur émettant un signal différent des deux premiers signaux, et
- un ou plusieurs marqueur(s), préférentiellement au moins deux marqueurs de CD de cellules plasmocytaires normales sélectionnés parmi les marqueurs de CD19, CD27, CD45, CD81, CD5L, CD11a, CD16b, CD24, CD36, CD52, CD68, CD79a, CD80, CD82, CD84, CD99 et CD163, préférentiellement les marqueurs de CD19, CD27, CD45 et CD81, plus préférentiellement un marqueur de CD19 et un marqueur de CD27, chacun de ces un ou plusieurs marqueur(s) émettant un seul et même signal autrement nommé 'signal détectable unique', différent des trois premiers signaux, et
- un ou plusieurs marqueur(s), préférentiellement au moins deux marqueurs, plus préférentiellement au moins trois marqueurs de CD de cellules plasmocytaires tumorales sélectionnés parmi les marqueurs de CD56, CD117, CD200, CD20, CD28, CD1D, CD2BP2, CD32c, CD47, CD59, CD109, CD229, CD300a et CD320, préférentiellement les marqueurs de CD56, CD117, CD200, CD20 et CD28, plus préférentiellement un marqueur de CD56, un marqueur de CD117 et un marqueur de CD200, chacun de ces un ou plusieurs marqueur(s) émettant un seul et même signal autrement nommé 'signal détectable unique', différent des quatre premiers signaux,
pour mettre en œuvre le procédé selon l'invention.

Avantageusement, la présente invention a pour objet l'utilisation d'une composition comprenant au moins :
- un marqueur de la chaîne κ et un marqueur de la chaîne λ, émettant un signal différent ; et
- un marqueur choisi parmi : un marqueur de CD38 et un marqueur de CD138, ledit marqueur émettant un signal différent des deux premiers signaux ; et
- un marqueur de CD19 et un marqueur de CD27, lesdits marqueurs émettant un seul et même signal autrement nommé 'signal détectable unique', différent des trois premiers signaux ; et
- un marqueur de CD56, un marqueur de CD117, un marqueur de CD200, lesdits marqueurs émettant un seul et même signal autrement nommé 'signal détectable unique', différent des quatre premiers signaux ;
pour mettre en œuvre le procédé selon l'invention.

Selon un mode de réalisation particulier, l'utilisation d'une composition selon l'invention comprend en outre un ou plusieurs marqueurs de la prolifération de plasmocytes tumoraux selon l'invention émettant un signal différent des signaux de chacun des autres marqueurs présents dans ladite composition.

La présente invention a également pour objet un kit pour mettre en œuvre le procédé selon l'invention, comprenant au moins :
- un marqueur de la chaîne κ, et un marqueur de la chaîne λ, émettant un signal différent, et
- un marqueur de CD38 ou un marqueur de CD138 émettant un signal différent des deux premiers signaux; et
- un ou plusieurs marqueur(s), préférentiellement au moins deux marqueurs de CD de cellules plasmocytaires normales sélectionnés parmi les marqueurs de CD19, CD27, CD45, CD81, CD5L, CD11a, CD16b, CD24, CD36, CD52, CD68, CD79a, CD80, CD82, CD84, CD99 et CD163, préférentiellement les marqueurs de CD19, CD27, CD45 et CD81, plus préférentiellement un marqueur de CD19 et un marqueur de CD27, chacun de ces un ou plusieurs marqueurs émettant un seul et même signal autrement nommé 'signal détectable unique', différent des trois premiers signaux ; et
- un ou plusieurs marqueur(s), préférentiellement au moins deux marqueurs, plus préférentiellement au moins trois marqueurs de CD de cellules plasmocytaires tumorales sélectionnés parmi les marqueurs de CD56, CD117, CD200, CD20, CD28, CD1D, CD2BP2, CD32c, CD47, CD59, CD109, CD229, CD300a et CD320, préférentiellement les marqueurs de CD56, CD117, CD200, CD20, et CD28, plus préférentiellement un marqueur de CD56, un marqueur de CD117 et un marqueur de CD200, chacun de ces un ou plusieurs marqueurs émettant un seul et même signal autrement nommé 'signal détectable unique', différent des quatre premiers signaux.

Selon un mode de réalisation préféré, le kit pour mettre en œuvre le procédé selon l'invention, comprend au moins :
- un marqueur de la chaîne κ et un marqueur de la chaîne λ, émettant un signal différent; et
- un marqueur de CD38 ou un marqueur de CD138, émettant un signal différent des deux premiers signaux; et
- un marqueur de CD19 et un marqueur de CD27, émettant un seul et même signal autrement nommé 'signal détectable unique', différent du des trois premiers signaux; et
- un marqueur de CD56, un marqueur de CD117 et un marqueur de CD200, émettant un seul et même signal autrement nommé 'signal détectable unique', différent des quatre premiers signaux.

Selon un mode de réalisation particulier, le kit selon l'invention comprend en outre un ou plusieurs marqueurs de la prolifération de plasmocytes tumoraux selon l'invention émettant un signal différent de chacun des autres marqueurs présents dans ledit kit.

Avantageusement, le kit selon l'invention comprend également au moins un marqueur de prolifération cellulaire choisi parmi:
- des analogues de bases de type BrdU et un anticorps anti-BrdU conjugué à l'APC ou EdU et un azoture couplé à un fluorochrome, préférentiellement l'Alexa Fluor® 647; et/ou
- des marqueurs de protéines qui sont présentes en cycle cellulaire actif, par exemple Ki67 reconnu par un anticorps anti-Ki67 conjugué à un fluorochrome, préférentiellement l'Alexa Fluor 647; et/ou
- des marqueurs d'acides désoxyribonucléiques (ADN), tel que le DAPI, PI, colorant Hoeschst (33342 ou 33258), etc...
chacun du ou desdits marqueur(s) de prolifération cellulaire émettant un signal différent des signaux émis par les autres marqueurs présents dans ledit kit, en particulier différent des cinq premiers signaux.

Selon un mode de réalisation préférentiel, le kit selon l'invention est caractérisé en ce qu'il comprend au moins un marqueur de prolifération cellulaire choisi parmi:
- un analogue de bases BromodesoxyUrydine (BrdU) ;
- une désoxyribonucléase (DNase, BrdU FlowKit ; BD Pharmingen ; Cat.: 557892) ;
- un marqueur comprenant un anticorps anti-BrdU conjugué à un fluorochrome, émettant un signal différent de chacun des autres signaux émis par les marqueurs présents dans ledit kit ;
- optionnellement du DAPI émettant un signal différent des signaux de chacun des autres marqueurs présents dans ledit kit ;
chacun du ou desdits marqueur(s) de prolifération cellulaire émettant un signal différent du signal de chacun des marqueurs présents dans ledit kit.

Selon un mode de réalisation préférentiel, le kit selon l'invention est caractérisé en ce qu'il comprend au moins un marqueur de prolifération cellulaire choisi parmi:
- un analogue de bases Ethynyldésoxyuridine (EdU) ;
- un azoture couplé à un fluorochrome, émettant un signal différent de chacun des autres signaux émis par les marqueurs présents dans ledit kit;
- optionnellement du DAPI émettant un signal différent des signaux de chacun des marqueurs présents dans ledit kit.

D'autres caractéristiques et avantages de l'invention apparaissent dans les exemples et figures suivants :

### DESCRIPTION DES FIGURES

Figure 1 : Discrimination des plasmocytes tumoraux par fenêtrages successifs.
Figure 2 : Représentation de la discrimination des plasmocytes tumoraux par les fenêtrages successifs selon la méthode STI.
Figure 3 : Suivi de la prolifération des plasmocytes normaux et tumoraux par l'utilisation de BrdU et DAPI ou de Ki67 et DAPI à partir d'un échantillon de patient atteint de myélome multiple avec un taux normal de plasmocytes en phase S.
Figure 4 : Suivi de la prolifération des plasmocytes normaux et tumoraux par l'utilisation de BrdU et DAPI ou de Ki67 et DAPI à partir d'un échantillon de patient atteint de myélome multiple avec un fort taux de plasmocytes en phase S.
Figure 5 : Détermination du pourcentage de cellules tumorales en phase S par l'utilisation de DAPI et d'EdU.

### EXEMPLES

### Exemple 1 : Détection des cellules plasmocytaires tumorales et normales chez un patient atteint de MM

Cette stratégie a pour principe la discrimination des cellules d'intérêt grâce à leur taille, leur complexité structurale et la présence des marqueurs cellulaires, ou antigènes, reconnus par des anticorps couplés à des fluorochromes. Elle compte 8 antigènes caractéristiques des cellules plasmocytaires (CD38, κ, λ CD19, CD27, CD56, CD117 et CD200) dont les anticorps spécifiques sont couplés à 5 fluorochromes différents dans un unique tube (CD38/PeCy5.5, κ/PECF594, λ/FITC, (CD19, CD27)/PeCy7, (CD56, CD117, CD200)/PE). Cette stratégie peut être réalisée en une demi-journée.

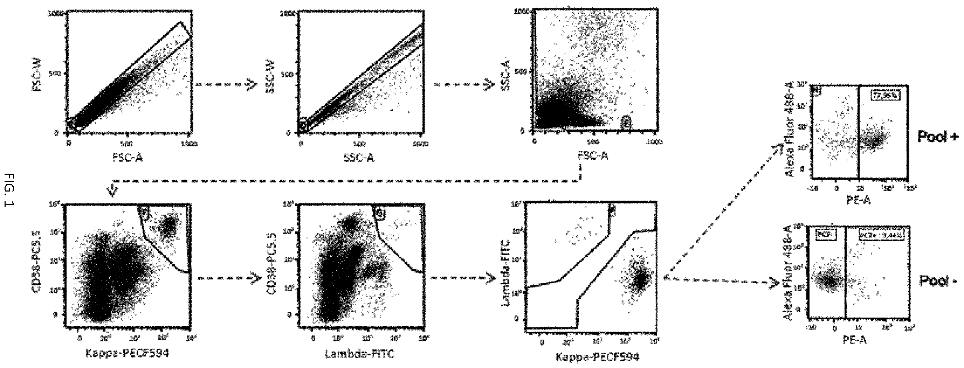

Les anticorps anti-CD38, anti-kappa et anti-lambda sont utilisés pour discriminer les plasmocytes totaux des autres leucocytes et deux groupes d'anticorps permettent l'identification des cellules tumorales. Le groupe négatif contient les anticorps anti-CD19 et anti-CD27 et le groupe positif contient les anticorps anti-CD56, anti-CD117 et anti-CD200. La Figure 1 représente les différents fenêtrages réalisés pour identifier les plasmocytes. La première étape consiste à éliminer les multiplets grâce à l'analyse de forme des impulsions sur les canaux FSC et SSC. Puis, les débris sont éliminés sur la matrice FSC/SSC. Les cellules qui expriment fortement soit CD38 et κ, soit CD38 et λ sont alors sélectionnées sur les matrices CD38/κ ou CD38/λ respectivement. Les événements sur la diagonale de la matrice κ/λ, sont éliminés. Après avoir discriminé la population de plasmocytes, les cellules tumorales sont identifiées grâce aux groupes positif et négatif.

### Préparation de l'échantillon

Suite au prélèvement de la moelle osseuse, cette dernière doit être filtrée afin d'éliminer les éventuels débris d'os. Les globules rouges sont ensuite lysés avec une lyse NH₄Cl (ratio ¼) et leurs débris sont en grande partie éliminés par centrifugation. Les anticorps membranaires couplés à leur fluorochrome respectif CD56-PE (Beckman Coulter ; Cat. : A07788), CD117-PE (Beckman Coulter ; Cat. : IM2732), CD200-PE (BD Pharmingen ; Cat. : 552475) (groupe positif), CD19-PeCy7 (Beckman Coulter ; Cat. : IM3628), CD27-PeCy7 (Beckman Coulter ; Cat. : A54823) (groupe négatif), CD38-PeCy5.5 (Beckman Coulter ; Cat. : A70205) sont déposés directement dans la préparation cellulaire selon les recommandations du fabricant. Après une incubation de 20 minutes à 4°C et un lavage au PBS, les cellules sont fixées (kit Intraprep ; Beckman Coulter ; Cat. : A07803). Ces dernières sont ensuite lavées avec du PBS pour éliminer la totalité de la solution de fixation. Les cellules sont enfin perméabilisées (kit Intraprep ; Beckman Coulter ; Cat. : A07803) et marquées avec les couples anticorps intracytoplasmiques/fluorochromes κ-PE-CF594 (BD Pharmingen ; Cat. : 562620) et λ-FITC (BD Pharmingen ; Cat. : 555796) selon les recommandations du fabricant. Après incubation de 20 minutes à 4°C et lavage au PBS, l'échantillon est analysé grâce au dispositif de cytométrie en flux (BD LSRFORTESSA X-20, BD Biosciences).

### Analyse

### Elimination des multiplets

Dans un premier temps, les cellules qui passent au même instant dans la fenêtre de mesure sont éliminées en utilisant les paramètres FSC et SSC qui correspondent respectivement à la taille et à la complexité structurale de la cellule. Pour ces deux paramètres, la matrice de la hauteur et de l'aire du pic de mesure est utilisée et les événements sortant de la diagonale de cette matrice sont éliminés. En effet, les valeurs de la hauteur et de l'aire sont proportionnelles et l'absence de cette proportionnalité (points à l'extérieur de la diagonale) traduit la présence de doublets voire multiplets.

### Elimination des débris

On élimine ensuite les débris cellulaires dus à la lyse, principalement les débris de globules rouges. La matrice FSC/SSC est utilisée pour sélectionner l'ensemble des leucocytes et éliminer les débris cellulaires ayant une petite taille et une structure peu complexe.

### Sélection des plasmocytes totaux

La sélection des plasmocytes totaux est réalisée grâce à trois marqueurs, permettant de détecter le CD38 et les deux protéines intracytoplasmiques κ et λ. Les plasmocytes sont des cellules exprimant fortement sur la surface de leur membrane le marqueur CD38. Ces cellules sont capables de produire des anticorps composés de chaines légères (κ ou λ) et de chaines lourdes. Chaque plasmocyte ne peut produire qu'un seul type de chaines légères et la proportion des plasmocytes dits « kappa » ou « lambda » est de 2/3 et 1/3 respectivement. Chez les patients atteints de myélome multiple, la prolifération d'un clone κ ou λ va induire un déséquilibre du ratio κ/λ.

Les plasmocytes sont sélectionnés sur les matrices CD38/κ et CD38/λ indépendamment.

### Elimination des événements κ/λ

Les événements se trouvant sur la diagonale des dimensions κ et λ sont éliminés. En effet, les plasmocytes peuvent sécréter soit la chaîne légère κ soit la chaîne légère λ tout au long de leur vie mais jamais les deux.

### Discrimination plasmocytes tumoraux/normaux

Une fois la sélection des plasmocytes totaux réalisée, les plasmocytes tumoraux doivent être discriminés par rapport aux plasmocytes normaux en sélectionnant les cellules exprimant un marqueur du groupe positif (CD56/CD117/CD200) mesuré sur le canal du PE et/ou ayant une perte d'expression d'un des marqueurs du groupe négatif (CD19/CD27) mesurée sur le canal PE-Cy7.

### Exemple 2 : Comparaison de la stratégie selon l'invention avec une stratégie connue de l'état de l'art

La stratégie développée et mise en œuvre par le laboratoire "Suivi des thérapie innovante" et décrite dans l'état de l'art (Caraux et al. ; 2012), que nous appellerons ici "stratégie STI", fait appel à 10 anticorps dirigés contre des antigènes caractéristiques des cellules plasmocytaires (CD38, CD45, CD19, CD20, κ, λ, CD27, CD56, CD117 et CD200) couplés à 7 fluorochromes différents répartis dans 4 tubes indépendants.

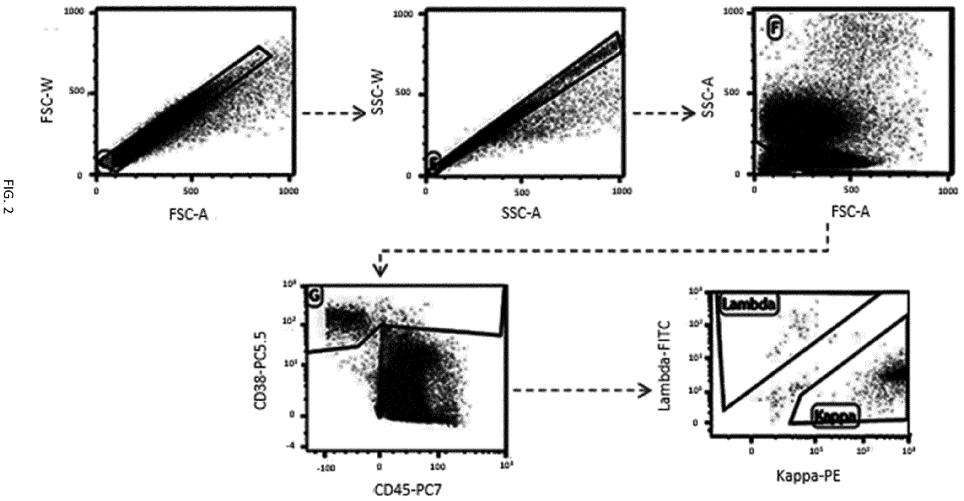

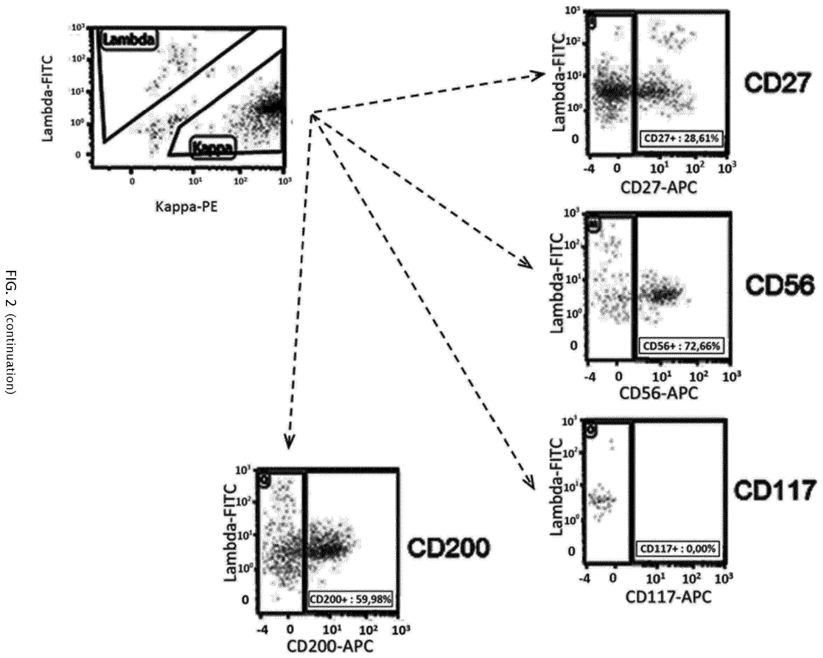

La Figure 2 représente les fenêtrages successifs de la stratégie STI. La première étape consiste à éliminer les multiplets (plusieurs cellules passant dans la fenêtre de détection en même temps) grâce aux dimensions FSC et SSC. Puis, les débris, de très petites tailles (FSC) et granulosités (SSC) sont éliminés sur la matrice FSC/SSC. Toutes les cellules exprimant fortement CD38, telles que les plasmocytes, sont alors sélectionnées sur le cytogramme CD38/CD45. Les événements sur la diagonale du cytogramme κ/λ sont alors éliminés. En effet, les plasmocytes sécrètent soit la chaîne légère κ, soit la chaîne légère λ. Après avoir identifié la population de plasmocytes totaux dans chacun des 4 tubes, les cellules tumorales sont différenciées des plasmocytes normaux grâce aux marqueurs permettant de détecter le CD27, le CD56, le CD117 et le CD200 par perte d'expression du premier et/ou expression des suivants. Cette méthode de l'état de l'art utilise donc sept couleurs, autant de détecteurs, deux lasers et quatre à cinq tubes, donc un nombre important et onéreux de dispositifs et de réactifs. En outre l'utilisation d'une telle méthode nécessite les compétences d'un expert en cytométrie pour la préparation, l'acquisition, la compensation et l'analyse des données. Comparativement, la méthode selon l'invention (exemple 1) est plus simple à mettre en œuvre et plus économique : elle peut se faire avec cinq détecteurs, un seul laser et un seul tube, et ne nécessite pas l'intervention d'un expert en cytométrie pour l'interprétation des résultats.

### Exemple 3. Détermination du pourcentage de cellules tumorales en phase S

L'analyse BrdU permet de déterminer le pourcentage de cellules tumorales en phase S (phase de Synthèse). Le DAPI (4',6'-diamidino-2-phényllindole, molécule fluorescente capable de se lier fortement aux bases Adénine (A) et Thymidine (T) de l'ADN) est utilisé pour permettre de quantifier l'ADN (2N/4N).

### Matériels et méthodes

Pour suivre la prolifération cellulaire, le kit APC BrdU Flow Kit a été utilisé selon les recommandations du fournisseur (BD Pharmigen, Cat. : 557892).

A la suite du protocole décrit dans l'exemple 1, 350µL d'un mélange DAPI/PermWash à 2µL/mL ont été ajoutés à la solution cellulaire puis incubé 20 minutes à 4°C. La solution cellulaire est ensuite lavée avec du PBS puis reprise dans du PBS avant d'être analysée en cytométrie en flux (cytomètre CyAn™ ADP - Beckman Coulter).

### Résultats

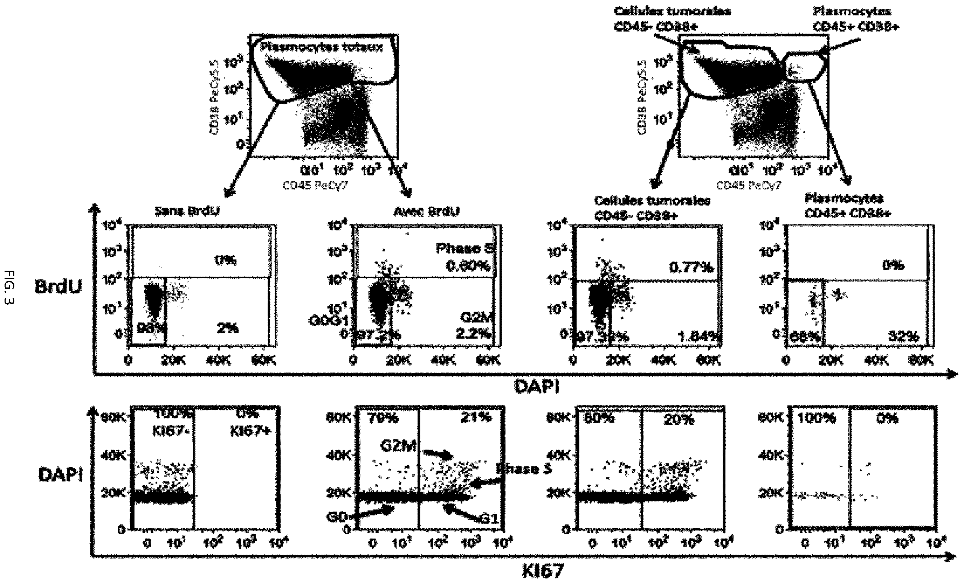

Patient atteint de myélome multiple avec un taux normal de plasmocytes en phase S (0,60%) (Figure 3).

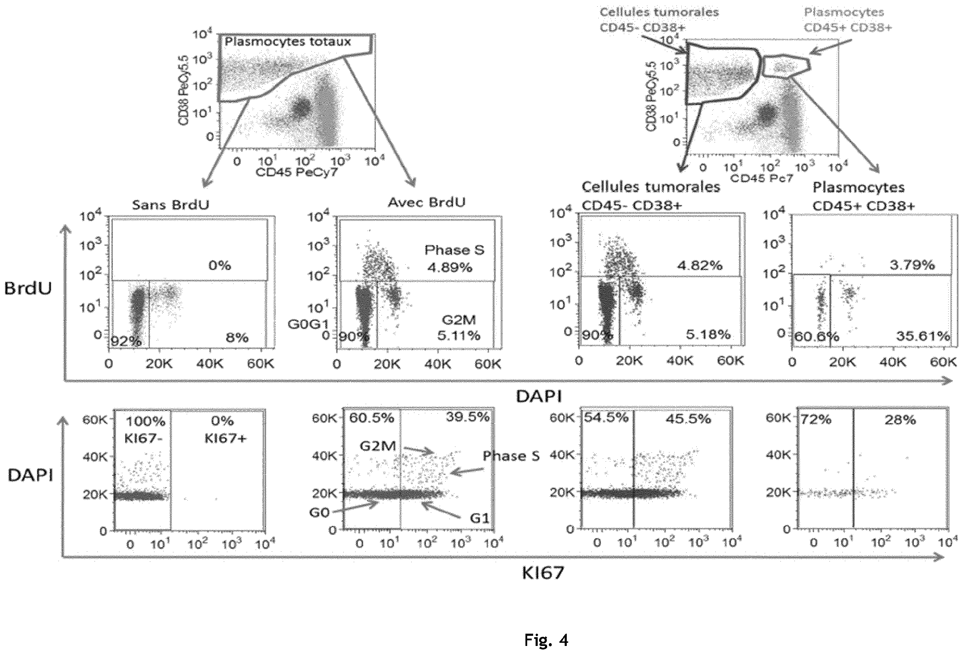

Patient atteint de myélome multiple avec un fort taux de plasmocytes en phase S (4,89%) (Figure 4).

### Exemple 4. Détermination du pourcentage de cellules engagées dans le cycle cellulaire (non quiescentes)

Le Ki67 permet de quantifier le pourcentage de cellules engagées dans le cycle cellulaire (non quiescentes). Le DAPI (4',6'-diamidino-2-phényllindole, molécule fluorescente capable de se lier fortement aux bases Adénine (A) et Thymidine (T) de l'ADN) est utilisé pour permettre de quantifier l'ADN (2N/4N).

### Matériels et méthodes

Deux tubes sont utilisés, un contrôle négatif avec l'isotype APC (Beckman Coulter, Ref: IM2475U) ajouté à la solution cellulaire au moment du marquage des anticorps de surface et un tube Ki67 positif avec l'anti-Ki67 couplé à l'Alexa Fluor 647 (BD Pharmigen, Cat. : 558615) ajouté à la solution cellulaire au moment du marquage des anticorps intracytoplasmiques.

A la suite du précédent protocole, 350µL d'un mélange DAPI/PermWash à 2µL/mL ont été ajoutés à la solution cellulaire puis incubé 20 minutes à 4°C. La solution cellulaire est ensuite lavé avec du PBS puis reprise dans du PBS avant d'être analysée en cytométrie en flux (cytomètre CyAn™ ADP - Beckman Coulter).

### Résultats

Patient atteint de myélome multiple avec un taux de cellules engagées dans le cycle cellulaire (non quiescentes) (21%) (Figure 3).

Patient atteint de myélome multiple avec un taux de cellules engagées dans le cycle cellulaire (non quiescentes) (39,5%) (Figure 4).

### Exemple 5. Détermination du pourcentage de cellules tumorales en phase S par l'utilisation de DAPI et d'EdU

### Matériels et méthodes

Pour suivre la prolifération cellulaire, le kit Click it® Plus EdU Alexa Fluor® 647 Flow Cytometry Assay Kit a été utilisé selon les recommandations du fournisseur (Life Technologies, Cat. : C10635).

A la suite du précédent protocole, 350µL d'un mélange DAPI/PermWash à 2µL/mL ont été ajoutés à la solution cellulaire puis incubé 20 minutes à 4°C. La solution cellulaire est ensuite lavé avec du PBS puis reprise dans du PBS avant d'être analysée en cytométrie en flux (cytomètre BD LSRFortessa™X-20 - BD Biosciences).

### Résultats

Le suivi de la prolifération des plasmocytes peut être réalisé en deux dimensions (DAPI vs EdU) ou en une dimension (histogramme EdU).

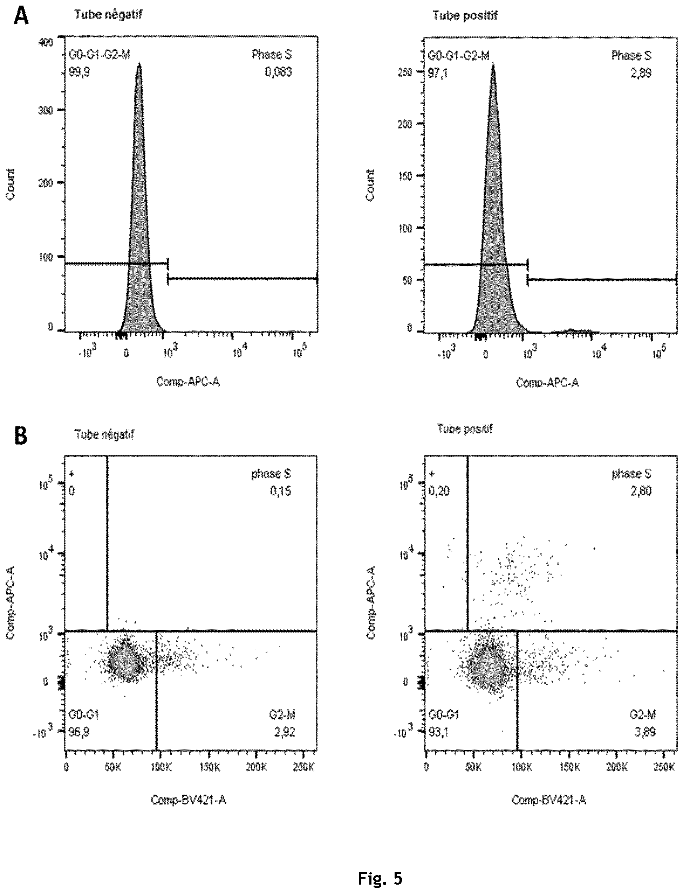

Les résultats présentés en figure 5 montrent qu'en utilisant une ou deux dimensions il est possible d'identifier respectivement 2,89% et 2,80% de plasmocytes en phase S en présence d'EdU (Tube positif) chez un patient diagnostiqué avec un myélome multiple.

### REFERENCES BIBLIOGRAPHIQUES

Caraux, A., Klein, B., Paiva, B., Bret, C., Schmitz, A., Fuhler, G.M., Bos, N.A., Johnsen, H.E., Orfao, A., Perez-Andres, M., et al. (2010). Circulating human B and plasma cells. Age-associated changes in counts and detailed characterization of circulating normal CD138- and CD138+ plasma cells. Haematologica 95, 1016-1020.

Caraux, A., Vincent, L., Bouhya, S., Quittet, P., Moreaux, J., Requirand, G., Veyrune, J.-L., Olivier, G., Cartron, G., Rossi, J.-F., et al. (2012). Residual malignant and normal plasma cells shortly after high dose melphalan and stem cell transplantation. Highlight of a putative therapeutic window in Multiple Myeloma? Oncotarget 3, 1335-1347.

Martinez-Lopez, J., Lahuerta, J.J., Pepin, F., González, M., Barrio, S., Ayala, R., Puig, N., Montalban, M.A., Paiva, B., Weng, L., et al. (2014). Prognostic value of deep sequencing method for minimal residual disease detection in multiple myeloma. Blood 123, 3073-3079.

Mateo, G., Montalbán, M.A., Vidriales, M.-B., Lahuerta, J.J., Mateos, M.V., Gutiérrez, N., Rosiñol, L., Montejano, L., Bladé, J., Martínez, R., et al. (2008). Prognostic Value of Immunophenotyping in Multiple Myeloma: A Study by the PETHEMA/GEM Cooperative Study Groups on Patients Uniformly Treated With High-Dose Therapy. J. Clin. Oncol. 26, 2737-2744. Paiva, B., Vidriales, M.-B., Cerveró, J., Mateo, G., Pérez, J.J., Montalbán, M.A., Sureda, A., Montejano, L., Gutiérrez, N.C., Coca, A.G. de, et al. (2008). Multiparameter flow cytometric remission is the most relevant prognostic factor for multiple myeloma patients who undergo autologous stem cell transplantation. Blood 112, 4017-4023.

Paiva, B., Vídriales, M.-B., Montalbán, M.-Á., Pérez, J.J., Gutiérrez, N.C., Rosiñol, L., Martínez-López, J., Mateos, M.-V., Cordón, L., Oriol, A., et al. (2012). Multiparameter flow cytometry evaluation of plasma cell DNA content and proliferation in 595 transplant-eligible patients with myeloma included in the Spanish GEM2000 and GEM2005<65y trials. Am. J. Pathol. 181, 1870-1878.

Paiva, B., Chandia, M., Puig, N., Vidriales, M.-B., Perez, J.J., Lopez-Corral, L., Ocio, E.M., Garcia-Sanz, R., Gutierrez, N.C., Jimenez-Ubieto, A., et al. (2014). The prognostic value of multiparameter flow cytometry minimal residual disease assessment in relapse multiple myeloma. Haematologica.

Rawstron, A.C., Child, J.A., de Tute, R.M., Davies, F.E., Gregory, W.M., Bell, S.E., Szubert, A.J., Navarro-Coy, N., Drayson, M.T., Feyler, S., et al. (2013). Minimal residual disease assessed by multiparameter flow cytometry in multiple myeloma: impact on outcome in the Medical Research Council Myeloma IX Study. J. Clin. Oncol. Off. J. Am. Soc. Clin. Oncol. 31, 2540-2547.

## Revendications

1. Procédé de détection par cytométrie en flux de la présence de cellules plasmocytaires normales et de cellules plasmocytaires tumorales dans un échantillon de cellules d'un patient, ledit procédé comprenant les étapes suivantes:
a) la mise en contact dudit échantillon de cellules avec :
- un marqueur de la chaîne κ un marqueur de la chaîne λ, et un marqueur choisi parmi :
un marqueur de CD38 et un marqueur de CD138, chacun des trois marqueurs émettant un signal différent (ci-après les 'trois premiers signaux'), et
- un ou plusieurs marqueur(s) de cellules plasmocytaires tumorales, chacun de ces un ou plusieurs marqueur(s) émettant un seul et même signal autrement nommé 'signal détectable unique' (ci-après le 'quatrième signal'), différent desdits trois premiers signaux, et
- un ou plusieurs marqueur(s) de CD de cellules plasmocytaires normales, chacun de ces un ou plusieurs marqueur(s) émettant un seul et même signal autrement nommé 'signal détectable unique' (ci-après le 'cinquième signal'), différent des quatre premiers signaux,
b) la détection des cellules plasmocytaires **caractérisées par** :
- un signal positif avec un marqueur de CD38 ou un marqueur de CD138, et
- un signal positif avec un marqueur de la chaîne κ ou un marqueur de la chaîne λ,
c) la détection des cellules plasmocytaires tumorales parmi les cellules plasmocytaires détectées à l'étape b) présentant au moins un des critères suivants:
- signal positif avec au moins un marqueur spécifique de cellules plasmocytaires tumorales, et/ou
- un signal négatif ou significativement réduit avec au moins un marqueur de cellules plasmocytaires normales,
d) l'analyse des résultats obtenus lors des étapes b) et c) et la détermination de la présence de cellules plasmocytaires normales et de cellules plasmocytaires tumorales.

2. Procédé selon la revendication 1, **caractérisé en ce que** :
- les CD spécifiques de cellules plasmocytaires normales du procédé selon l'invention sont choisis parmi : CD19, CD27, CD45, CD81, CD5L, CD11a, CD16b, CD24, CD36, CD52, CD68, CD79a, CD80, CD82, CD84, CD99 et CD163, préférentiellement CD19, CD27, CD45 et CD81 plus préférentiellement CD19, CD27, et
- les CD spécifiques de cellules plasmocytaires tumorales du procédé selon l'invention sont choisis parmi : CD56, CD117, CD200, CD20, CD28, CD1D, CD2BP2, CD32c, CD47, CD59, CD109, CD229, CD300a et CD320, préférentiellement CD56, CD117, CD200, CD20, et CD28, plus préférentiellement CD56, CD117, CD200.

3. Procédé selon l'une quelconque des revendications précédentes, dans lequel le marqueur CD38 ou CD138 est respectivement un anticorps anti-CD38 ou anti-CD138 conjugué au fluorochrome PE-Cy5.5, le marqueur de la chaîne κ est l'anticorps anti-chaîne κ conjugué au fluorochrome PE-CF594 et le marqueur de la chaîne λ est un anticorps anti-chaîne λ conjugué au fluorochrome FITC.

4. Procédé selon la revendication 2, **caractérisé en ce que** les marqueurs CD19 et CD27 sont des anticorps anti-CD19 et anti-CD27 conjugués au fluorochrome PE-Cy7, et les marqueurs CD56, CD117 et CD200 sont des anticorps anti-CD56, anti-CD117 et anti-CD200 conjugués au fluorochrome PE.

5. Procédé selon l'une quelconque des revendications 1 à 4, comprenant en outre la détermination de l'index de prolifération des plasmocytes tumoraux comprenant les étapes suivantes :
A. mise en contact dudit échantillon de cellules avec un ou plusieurs marqueur(s), préférentiellement deux marqueurs, de prolifération cellulaire, émettant un signal différent des autres marqueurs utilisés;
B. détection des plasmocytes tumoraux en prolifération par analyse du signal émis par ledit ou lesdits marqueur(s);
C. détermination du pourcentage de cellules tumorales et de plasmocytes normaux étant en phase S ;
D. optionnellement détermination de la diploïdie (G0/G1 ou G2/M) lorsque au moins un du ou desdits marqueur(s) de prolifération cellulaire est un marqueur de l'ADN.

6. Procédé selon l'une quelconque des revendications 1 à 5, dans lequel l'échantillon de cellules est choisi parmi des cellules de la moelle osseuse, du sang, du sérum, d'un extrait sanguin, des PBMCs, du liquide céphalorachidien, du liquide pleural et des ganglions lymphatiques, et préférentiellement de la moelle osseuse d'un patient.

7. Procédé selon l'une quelconque des revendications 1 à 6, dans lequel ledit patient est atteint de gammapathie monoclonale.

8. Procédé de diagnostic *in vitro* ou de suivi de gammapathie monoclonale, à partir d'un échantillon de cellules d'un patient comprenant les étapes suivantes :
1) la mise en œuvre d'un procédé selon l'une des revendications 1 à 7, et
2) mise en évidence d'un risque de développer une gammapathie monoclonale :
a) si la proportion de cellules plasmocytaires tumorales parmi les cellules leucocytaires totales, d'après les résultats de l'analyse de l'étape d) du procédé selon l'invention, dépasse un seuil prédéfini à partir de résultats obtenus par le procédé selon l'invention sur des sujets contrôle sains; et/ou
b) si le nombre de plasmocytes tumoraux par unité de volume dépasse un seuil prédéfini.

9. Procédé de suivi *in vitro* de l'évolution de la maladie d'un patient atteint d'une gammapathie monoclonale, à partir d'un premier et d'un second échantillon de cellules du patient, comprenant les étapes suivantes :
i) la mise en œuvre d'un procédé de détection selon l'une des revendications 1 à 7 dans un premier échantillon de cellules du patient,
ii) la mise en œuvre d'un procédé de détection selon l'une des revendications 1 à 7 dans un second échantillon de cellules du patient, ledit second échantillon ayant été prélevé après le prélèvement dudit premier échantillon, et
iii) la comparaison de l'analyse des résultats de l'étape d) obtenus avec le premier et le second échantillon, et
iv) le suivi de l'évolution de ladite gammapathie monoclonale dudit patient d'après les résultats de l'analyse de l'étape iii).

10. Composition comprenant au moins :
- un marqueur de la chaîne κ et un marqueur de la chaîne λ, émettant chacun un signal différent, et
- un marqueur choisi parmi : un marqueur de CD38 ou un marqueur de CD138, ledit marqueur émettant un signal différent des deux premiers signaux, et
- un ou plusieurs marqueur(s), préférentiellement au moins deux marqueurs, de CD spécifiques de cellules plasmocytaires normales sélectionnés parmi les marqueurs de CD19, CD27, CD45, CD81, CD5L, CD11a, CD16b, CD24, CD36, CD52, CD68, CD79a, CD80, CD82, CD84, CD99 et CD163, préférentiellement les marqueurs de CD19, CD27, CD45 et CD81, plus préférentiellement un marqueur de CD19 et un marqueur de CD27, chacun de ces un ou plusieurs marqueur(s) émettant un seul et même signal autrement nommé 'signal détectable unique' (ci-après le 'quatrième signal'), différent des trois premiers signaux, et
- un ou plusieurs marqueur(s), préférentiellement au moins deux marqueurs, plus préférentiellement au moins trois marqueurs de CD spécifiques de cellules plasmocytaires tumorales sélectionnés parmi les marqueurs de CD56, CD117, CD200, CD20, CD28, CD1D, CD2BP2, CD32c, CD47, CD59, CD109, CD229, CD300a et CD320, préférentiellement les marqueurs de CD56, CD117, CD200, CD20, et CD28, plus préférentiellement un marqueur de CD56, un marqueur de CD117 et un marqueur de CD200, chacun de ces un ou plusieurs marqueur(s) émettant un seul et même signal autrement nommé 'signal détectable unique' (ci-après le 'cinquième signal'), différent des quatre premiers signaux.

11. Utilisation d'une composition comprenant au moins :
- un marqueur de la chaîne κ et un marqueur de la chaîne λ émettant un signal différent, et
- un marqueur choisi parmi : un marqueur de CD38 et un marqueur de CD138, ledit marqueur émettant un signal différent des deux premiers signaux, et
- un ou plusieurs marqueur(s), préférentiellement au moins deux marqueurs de CD de cellules plasmocytaires normales sélectionnés parmi les marqueurs de CD19, CD27, CD45, CD81, CD5L, CD11a, CD16b, CD24, CD36, CD52, CD68, CD79a, CD80, CD82, CD84, CD99 et CD163, préférentiellement les marqueurs de CD19, CD27, CD45 et CD81, plus préférentiellement un marqueur de CD19 et un marqueur de CD27, chacun de ces un ou plusieurs marqueur(s) émettant un seul et même signal autrement nommé 'signal détectable unique' (ci-après le 'quatrième signal'), différent des trois premiers signaux, et
- un ou plusieurs marqueur(s), préférentiellement au moins deux marqueurs, plus préférentiellement au moins trois marqueurs de CD de cellules plasmocytaires tumorales sélectionnés parmi les marqueurs de CD56, CD117, CD200, CD20, CD28, CD1D, CD2BP2, CD32c, CD47, CD59, CD109, CD229, CD300a et CD320, préférentiellement les marqueurs de CD56, CD117, CD200, CD20, et CD28, plus préférentiellement un marqueur de CD56, un marqueur de CD117 et un marqueur de CD200, chacun de ces un ou plusieurs marqueur(s) émettant un seul et même signal autrement nommé 'signal détectable unique' (ci-après le 'cinquième signal'), différent des quatre premiers des signaux,
pour mettre en œuvre le procédé selon l'une des revendications 1 à 9.

12. Kit pour mettre en œuvre le procédé selon l'une quelconque des revendications 1 à 9, comprenant au moins :
- un marqueur de la chaîne κ, et un marqueur de la chaîne λ, émettant chacun un signal différent ; et
- un marqueur de CD38 ou un marqueur de CD138 émettant un signal différent des deux premiers signaux; et
- un ou plusieurs marqueur(s), préférentiellement au moins deux marqueurs de CD de cellules plasmocytaires normales sélectionnés parmi les marqueurs de CD19, CD27, CD45, CD81, CD5L, CD11a, CD16b, CD24, CD36, CD52, CD68, CD79a, CD80, CD82, CD84, CD99 et CD163, préférentiellement les marqueurs de CD19, CD27, CD45 et CD81, plus préférentiellement un marqueur de CD19 et un marqueur de CD27, chacun de ces un ou plusieurs marqueur(s) émettant un seul et même signal autrement nommé 'signal détectable unique' (ci-après le 'quatrième signal'), différent des trois premiers signaux; et
- un ou plusieurs marqueur(s), préférentiellement au moins deux marqueurs, plus préférentiellement au moins trois marqueurs de CD de cellules plasmocytaires tumorales sélectionnés parmi les marqueurs de CD56, CD117, CD200, CD20, CD28, CD1D, CD2BP2, CD32c, CD47, CD59, CD109, CD229, CD300a et CD320, préférentiellement les marqueurs de CD56, CD117, CD200, CD20, et CD28, plus préférentiellement un marqueur de CD56, un marqueur de CD117 et un marqueur de CD200, chacun de ces un ou plusieurs marqueur(s) émettant un seul et même signal autrement nommé 'signal détectable unique' (ci-après le 'cinquième signal'), différent des quatre premiers signaux.

13. Kit selon la revendication 12, **caractérisé en ce qu'**il comprend au moins un marqueur de prolifération cellulaire sélectionné parmi:
- des analogues de bases de type BrdU et un anticorps anti-BrdU conjugué à l'APC ou EdU et un azoture couplé à un fluorochrome, préférentiellement l'Alexa Fluor® 647 ; et/ou
- des marqueurs de protéines du cycle cellulaire actif, par exemple un anticorps anti-Ki67 conjugué à un fluorochrome, préférentiellement l'Alexa Fluor 647® ; et/ou
- des marqueurs d'acides désoxyribonucléiques (ADN), tel que le DAPI, PI, colorant Hoeschst (33342 ou 33258),
chacun du ou desdits marqueur(s) de prolifération cellulaire émettant un signal différent des signaux émis par les autres marqueurs présents dans ledit kit, en particulier différent des cinq premiers signaux.

## Patentansprüche

1. Verfahren zur Erkennung des Vorhandenseins von normalen Plasmazellen und von Tumorplasmazellen in einer Zellprobe eines Patienten durch Durchflusszytometrie, wobei das Verfahren die folgenden Schritte umfasst:
a) das Inkontaktbringen der Zellprobe mit:
- einem Marker der κ-Kette und einem Marker der λ-Kette und einem Marker, der ausgewählt wird aus: einem CD38-Marker und einem CD138-Marker, wobei jeder der drei Marker ein unterschiedliches Signal emittiert (nachfolgend die "drei ersten Signale"), und
- einem oder mehreren Markern für Tumorplasmazellen, wobei jeder von diesem/diesen einen oder mehreren Markern ein und dasselbe Signal emittiert, das anders auch als "eindeutiges detektierbares Signal" bezeichnet wird (nachfolgend das "vierte Signal"), das sich von den drei ersten Signalen unterscheidet, und
- einem oder mehreren CD-Markern für normale Plasmazellen, wobei jeder von diesem einen oder diesen mehreren Markern ein und dasselbe Signal emittiert, das anders auch als "eindeutiges detektierbares Signal" bezeichnet wird (nachfolgend das "fünfte Signal"), das sich von den vier ersten Signalen unterscheidet,
b) das Erkennen der Plasmazellen, die **gekennzeichnet sind durch**:
- ein positives Signal mit einem CD38-Marker oder einem CD138-Marker, und
- ein positives Signal mit einem Maker der κ-Kette oder einem Marker der λ-Kette,
c) das Erkennen von Tumorplasmazellen unter den im Schritt b) erkannten Plasmazellen, die mindestens eines der folgenden Kriterien aufweisen:
- positives Signal mit mindestens einem spezifischen Marker für Tumorplasmazellen, und/oder
- ein negatives oder wesentlich vermindertes Signal mit mindestens einem Marker für normale Plasmazellen,
d) das Analysieren der in den Schritten b) und c) erhaltenen Ergebnisse und die Bestimmung des Vorhandenseins von normalen Plasmazellen und von Tumorplasmazellen.

2. Verfahren nach Anspruch 1, **dadurch gekennzeichnet, dass**:
- die für normale Plasmazellen spezifischen CD des erfindungsgemäßen Verfahrens ausgewählt werden aus: CD19, CD27, CD45, CD81, CD5L, CD11a, CD16b, CD24, CD36, CD52, CD68, CD79a, CD80, CD82, CD84, CD99 und CD163, vorzugsweise CD19, CD27, CD45 und CD81, noch mehr zu bevorzugen CD19, CD27, und
- die für Tumorplasmazellen spezifischen CD des erfindungsgemäßen Verfahrens ausgewählt werden aus: CD56, CD117, CD200, CD20, CD28, CD1D, CD2BP2, CD32c, CD47, CD59, CD109, CD229, CD300a und CD320, vorzugsweise CD56, CD117, CD200, CD20 und CD28, noch mehr zu bevorzugen CD56, CD117, CD200.

3. Verfahren nach einem der vorhergehenden Ansprüche, wobei der CD38- oder CD138-Marker ein Anti-CD38- oder Anti-CD138-Antikörper ist, der mit dem Fluorochrom PE-Cy5.5 konjugiert ist, wobei der Marker der κ-Kette der Anti-κ-Kette-Antikörper ist, der mit dem Fluorochrom PE-CF594 konjugiert ist, und der Marker der λ-Kette ein Anti-λ-Kette-Antikörper ist, der mit dem Fluorochrom FITC konjugiert ist.

4. Verfahren nach Anspruch 2, **dadurch gekennzeichnet, dass** die CD19- und CD27-Marker Anti-CD19- und Anti-CD27-Antikörper sind, die mit dem Fluorochrom PE-Cy7 konjugiert sind, und die CD56-, CD117 und CD200-Marker Anti-CD56-, Anti-CD117- und Anti-CD200-Antikörper sind, die mit dem Fluorochrom PE konjugiert sind.

5. Verfahren nach einem der Ansprüche 1 bis 4, das ferner die Bestimmung des Proliferationsindex der Tumorplasmazellen umfasst, die die folgenden Schritte umfasst:
A. Inkontaktbringen der Zellprobe mit einem oder mehreren Markern, vorzugsweise zwei Markern, für die zelluläre Proliferation, die ein Signal emittieren, das sich von den anderen verwendeten Markern unterscheidet;
B. Erkennen der Tumorplasmazellen in Proliferation durch Analyse des von dem oder den Markern emittierten Signals;
C. Bestimmen des Prozentsatzes an Tumorzellen und an normalen Plasmazellen, die sich in der Phase S befinden;
D. optional Bestimmen der Diploidie (G0/G1 oder G2/M), wenn mindestens einer von dem oder den Markern für zelluläre Proliferation ein DNA-Marker ist.

6. Verfahren nach einem der Ansprüche 1 bis 5, wobei die Zellprobe ausgewählt wird aus Zellen von Knochenmark, Blut, Serum, einem Blutextrakt, PBMCs, Zerebrospinalflüssigkeit, Pleuraflüssigkeit und der Lymphknoten und vorzugsweise des Knochenmarks eines Patienten.

7. Verfahren nach einem der Ansprüche 1 bis 6, wobei der Patient von monoklonaler Gammopathie betroffen ist.

8. In-vitro-Verfahren zur Diagnose oder Verlaufskontrolle von monoklonaler Gammopathie ausgehend von einer Zellprobe eines Patienten, das die folgenden Schritte umfasst:
1) Durchführen eines Verfahrens nach einem der Ansprüche 1 bis 7, und
2) Nachweisen eines Risikos der Entwicklung einer monoklonalen Gammopathie:
a) wenn der Anteil an Tumorplasmazellen unter den Gesamtleukozyten nach den Ergebnissen der Analyse von Schritt d) des erfindungsgemäßen Verfahrens einen ausgehend von den durch das erfindungsgemäße Verfahren bei gesunden Kontrollpersonen erhaltenen Ergebnissen vordefinierten Schwellenwert überschreitet; und/oder
b) wenn die Anzahl an Tumorplasmazellen pro Volumeneinheit einen vorbestimmten Schwellenwert überschreitet.

9. *In-Vitro*-Verfahren zur Verlaufskontrolle der Erkrankung eines Patienten, der von einer monoklonalen Gammopathie betroffen ist, ausgehend von einer ersten und von einer zweiten Zellprobe des Patienten, das die folgenden Schritte umfasst:
i) Durchführen eines Verfahrens zur Erkennung nach einem der Ansprüche 1 bis 7 in einer ersten Zellprobe des Patienten,
ii) Durchführen eines Verfahrens zur Erkennung nach einem der Ansprüche 1 bis 7 in einer zweiten Zellprobe des Patienten, wobei die zweite Probe nach der Entnahme der ersten Probe entnommen wurde, und
iii) Vergleichen der Analyse der Ergebnisse von Schritt d), die mit der ersten und der zweiten Probe erhalten wurden, und
iv) Verlaufskontrolle der Entwicklung der monoklonalen Gammopathie des Patienten nach den Ergebnissen der Analyse von Schritt iii).

10. Zusammensetzung, die mindestens umfasst:
- einen Marker der κ-Kette und einen Marker der λ-Kette, die jeweils ein unterschiedliches Signal emittieren, und
- einen Marker, der ausgewählt ist aus: einem CD38-Marker oder einem CD138-Marker, wobei der Marker ein Signal emittiert, das sich von den zwei ersten Signalen unterscheidet, und
- einen oder mehrere Marker, vorzugsweise mindestens zwei Marker spezifischer CD normaler Plasmazellen, die ausgewählt sind aus den CD19-, CD27-, CD45-, CD81-, CD5L-, CD11a-, CD16b-, CD24-, CD36-, CD52-, CD68-, CD79a-, CD80-, CD82-, CD84-, CD99- und CD163-Markern, vorzugsweise den CD19-, CD27-, CD45- und CD81-Markern, noch mehr zu bevorzugen einem CD19-Marker und einem CD27-Marker, wobei jeder von dem einen oder den mehreren Markern ein und dasselbe Signal emittiert, das anders auch als "eindeutiges detektierbares Signal" (nachfolgend das "vierte Signal") bezeichnet wird, das sich von den drei ersten Signalen unterscheidet, und
- einen oder mehrere Marker, vorzugsweise mindestens zwei Marker, noch mehr zu bevorzugen mindestens drei spezifische CD-Marker für Tumorplasmazellen, die ausgewählt sind aus CD56-, CD117-, CD200-, CD20-, CD28-, CD1D-, CD2BP2-, CD32c-, CD47-, CD59-, CD109-, CD229-, CD300a- und CD320-Markern, vorzugsweise CD56-, CD117-, CD200-, CD20- und CD28-Markern, noch mehr zu bevorzugen einem CD56-Marker, einem CD117-Marker und einem CD200-Marker, wobei jeder von diesem einen oder diesen mehreren Markern ein und dasselbe Signal emittiert, das anders auch als "eindeutiges detektierbares Signal" (nachfolgend das "fünfte Signal") bezeichnet wird, das sich von den vier ersten Signalen unterscheidet.

11. Verwendung einer Zusammensetzung, die mindestens umfasst:
- einen Marker der κ-Kette und einen Marker der λ-Kette, die jeweils ein unterschiedliches Signal emittieren, und
- einen Marker, der ausgewählt ist aus: einem CD38-Marker und einem CD138-Marker, wobei der Marker ein Signal emittiert, das sich von den zwei ersten Signalen unterscheidet, und
- einen oder mehrere Marker, vorzugsweise mindestens zwei CD-Marker normaler Plasmazellen, die ausgewählt sind aus den CD19-, CD27-, CD45-, CD81-, CD5L-, CD11a-, CD16b-, CD24-, CD36-, CD52-, CD68-, CD79a-, CD80-, CD82-, CD84-, CD99- und CD163-Markern, vorzugsweise den CD19-, CD27-, CD45- und CD81-Markern, noch mehr zu bevorzugen einem CD19-Marker und einem CD27-Marker, wobei jeder von diesem einen oder diesen mehreren Markern ein und dasselbe Signal emittiert, das anders auch als "eindeutiges detektierbares Signal" (nachfolgend das "vierte Signal") bezeichnet wird, das sich von den drei ersten Signalen unterscheidet, und
- einen oder mehrere Marker, vorzugsweise mindestens zwei Marker, noch mehr zu bevorzugen mindestens drei CD-Marker für Tumorplasmazellen, die ausgewählt sind aus CD56-, CD117-, CD200-, CD20-, CD28-, CD1D-, CD2BP2-, CD32c-, CD47-, CD59-, CD109-, CD229-, CD300a- und CD320-Markern, vorzugsweise CD56-, CD117-, CD200-, CD20- und CD28-Markern, noch mehr zu bevorzugen einem CD56-Marker, einem CD117-Marker und einem CD200-Marker, wobei jeder von diesem einen oder diesen mehreren Markern ein und dasselbe Signal emittiert, das anders auch als "eindeutiges detektierbares Signal" (nachfolgend das "fünfte Signal") bezeichnet wird, das sich von den vier ersten Signalen unterscheidet,
zum Durchführen des Verfahrens nach einem der Ansprüche 1 bis 9.

12. Kit zum Durchführen des Verfahrens nach einem der Ansprüche 1 bis 9, das mindestens umfasst:
- einen Marker der κ-Kette und einen Marker der λ-Kette, die jeweils ein unterschiedliches Signal emittieren; und
- einen CD38-Marker oder einen CD138-Marker, der ein Signal emittiert, das sich von den zwei ersten Signalen unterscheidet; und
- einen oder mehrere Marker, vorzugsweise mindestens zwei CD-Marker normaler Plasmazellen, die ausgewählt sind aus den CD19-, CD27-, CD45-, CD81-, CD5L-, CD11a-, CD16b-, CD24-, CD36-, CD52-, CD68-, CD79a-, CD80-, CD82-, CD84-, CD99- und CD163-Markern, vorzugsweise den CD19-, CD27-, CD45- und CD81-Markern, noch mehr zu bevorzugen einem CD19-Marker und einem CD27-Marker, wobei jeder von diesem einen oder diesen mehreren Markern ein und dasselbe Signal emittiert, das anders auch als "eindeutiges detektierbares Signal" (nachfolgend das "vierte Signal") bezeichnet wird, das sich von den drei ersten Signalen unterscheidet; und
- einen oder mehrere Marker, vorzugsweise mindestens zwei Marker, noch mehr zu bevorzugen mindestens drei CD-Marker für Tumorplasmazellen, die ausgewählt sind aus CD56-, CD117-, CD200-, CD20-, CD28-, CD1D-, CD2BP2-, CD32c-, CD47-, CD59-, CD109-, CD229-, CD300a- und CD320-Markern, vorzugsweise CD56-, CD117-, CD200-, CD20- und CD28-Markern, noch mehr zu bevorzugen einem CD56-Marker, einem CD117-Marker und einem CD200-Marker, wobei jeder von diesem einen oder diesen mehreren Markern ein und dasselbe Signal emittiert, das anders auch als "eindeutiges detektierbares Signal" (nachfolgend das "fünfte Signal") bezeichnet wird, das sich von den vier ersten Signalen unterscheidet.

13. Kit nach Anspruch 12, **dadurch gekennzeichnet, dass** es mindestens einen Marker für zelluläre Proliferation umfasst, der ausgewählt ist aus:
- Basenanaloga des Typs BrdU und einem Anti-BrdU-Antikörper, der mit dem APC oder EdU konjugiert ist, und einem an ein Fluorochrom, vorzugsweise das Alexa Fluor® 647, gebundenen Azid; und/oder
- Proteinmarker des aktiven Zellzyklus, zum Beispiel ein Anti-Ki67-Antikörper, der mit einem Fluorochrom, vorzugsweise dem Alexa Fluor 647®, konjugiert ist; und/oder
- Desoxyribonukleinsäuremarkern (DNA) wie beispielsweise DAPI, PI, Höchst-Farbstoff (33342 oder 33258),
wobei jeder der Marker für zelluläre Proliferation ein Signal emittiert, das sich von den Signalen, die von den anderen in dem Kit vorhandenen Markern emittiert werden, insbesondere von den fünf ersten Signalen, unterscheidet.

## Claims

1. A process for detecting, by flow cytometry, the presence of normal plasma cells and of tumoral plasma cells in a sample of cells from a patient, said process comprising the following steps:
a) bringing said sample of cells into contact with:
- a κ chain marker, a λ chain marker, and a marker selected from: a CD38 marker and a CD138 marker, each of the three markers emitting a different signal (hereinafter the 'first three signals'), and
- one or more tumoral plasma cell marker(s), each of these one or more marker (s) emitting a single signal otherwise known as the 'single detectable signal' (hereinafter the 'fourth signal'), different than said first three signals, and
- one or more normal plasma cell CD marker (s), each of these one or more marker(s) emitting a single signal otherwise known as the 'single detectable signal' (hereinafter the 'fifth signal'), different than the first four signals,
b) detecting the plasma cells **characterized by**:
- a positive signal with a CD38 marker or a CD138 marker, and
- a positive signal with a κ chain marker or a λ chain marker,
c) detecting the tumoral plasma cells among the plasma cells detected in step b) having at least one of the following criteria:
- a positive signal with at least one marker specific for tumoral plasma cells, and/or
- a negative or significantly reduced signal with at least one marker for normal plasma cells,
d) analyzing the results obtained during steps b) and c) and determining the presence of normal plasma cells and of tumoral plasma cells.

2. The process as claimed in claim 1, **characterized in that**:
- the CDs specific for normal plasma cells of the process according to the invention are selected from: CD19, CD27, CD45, CD81, CD5L, CD11a, CD16b, CD24, CD36, CD52, CD68, CD79a, CD80, CD82, CD84, CD99 and CD163, preferentially CD19, CD27, CD45 and CD81, more preferentially CD19, CD27, and
- the CDs specific for tumoral plasma cell of the process according to the invention are selected from: CD56, CD117, CD200, CD20, CD28, CD1D, CD2BP2, CD32c, CD47, CD59, CD109, CD229, CD300a and CD320, preferentially CD56, CD117, CD200, CD20, and CD28, more preferentially CD56, CD117, CD200.

3. The process as claimed in any one of the preceding claims, wherein the CD38 or CD138 marker is respectively an anti-CD38 or anti-CD138 antibody conjugated to the PE-Cy5.5 fluorochrome, the κ chain marker is the anti-κ chain antibody conjugated to the PE-CF594 fluorochrome and the λ chain marker is an anti-λ chain antibody conjugated to the FITC fluorochrome.

4. The process as claimed in claim 2, **characterized in that** the CD19 and CD27 markers are anti-CD19 and anti-CD27 antibodies conjugated to the PE-Cy7 fluorochrome, and the CD56, CD117 and CD200 markers are anti-CD56, anti-CD117 and anti-CD200 antibodies conjugated to the PE fluorochrome.

5. The process as claimed in any one of claims 1 to 4, further comprising determining the proliferation index of the tumoral plasma cell, comprising the following steps:
A. bringing said sample of cells into contact with one or more, preferentially two, cell proliferation marker(s), emitting a signal different than the other markers used;
B. detecting the proliferating tumoral plasma cells by analysis of the signal emitted by said marker(s);
C. determining the percentage of tumoral cells and of normal plasma cells which are in the S phase;
D. optionally determining the diploidy (G0/G1 or G2/M) when at least one of said cell proliferation marker(s) is a DNA marker.

6. The process as claimed in any one of claims 1 to 5, wherein the sample of cells is selected from cells from the bone marrow, the blood, the serum, a blood extract, PBMCs, the cerebrospinal fluid, the pleural fluid and the lymph nodes, and preferentially from the bone marrow of a patient.

7. The process as claimed in any one of claims 1 to 6, wherein said patient is suffering from monoclonal gammopathy.

8. A process for diagnosing, *in vitro,* or monitoring monoclonal gammopathy, using a sample of cells from a patient, comprising the following steps:
1) carrying out the process as claimed in one of claims 1 to 7, and
2) demonstrating a risk of developing a monoclonal gammopathy:
a) if the proportion of tumoral plasma cells among the total leukocyte cells, according to the results of the analysis of step d) of the process according to the invention, exceeds a threshold predefined on the basis of results obtained by the process according to the invention on healthy control subjects; and/or
b) if the number of tumoral plasma cells per unit of volume exceeds a predefined threshold.

9. A process for monitoring, *in vitro,* the progression of the disease of a patient suffering from a monoclonal gammopathy, using a first and a second sample of cells from the patient, comprising the following steps:
i) carrying out the detection process as claimed in one of claims 1 to 7 in a first sample of cells from the patient,
ii) carrying out the detection process as claimed in one of claims 1 to 7 in a second sample of cells from the patient, said second sample having been taken after said first sample was taken, and
iii) comparing the analysis of the results of step d) obtained with the first and the second sample, and
iv) monitoring the progression of said monoclonal gammopathy of said patient according to the results of the analysis of step iii).

10. A composition comprising at least:
- a κ chain marker and a λ chain marker, each emitting a different signal, and
- a marker selected from: a CD38 marker or a CD138 marker, said marker emitting a signal different than the first two signals, and
- one or more CD marker (s), preferentially at least two CD markers, specific for normal plasma cells selected from CD19, CD27, CD45, CD81, CD5L, CD11a, CD16b, CD24, CD36, CD52, CD68, CD79a, CD80, CD82, CD84, CD99 and CD163 markers, preferentially CD19, CD27, CD45 and CD81 markers, more preferentially a CD19 marker and a CD27 marker, each of these one or more marker (s) emitting a single signal otherwise known as the 'single detectable signal' (hereinafter the 'fourth signal'), different than the first three signals, and
- one or more CD marker (s), preferentially at least two CD markers, more preferentially at least three CD markers specific for tumoral plasma cell selected from CD56, CD117, CD200, CD20, CD28, CD1D, CD2BP2, CD32c, CD47, CD59, CD109, CD229, CD300a and CD320 markers, preferentially CD56, CD117, CD200, CD20, and CD28 markers, more preferentially a CD56 marker, a CD117 marker, and a CD200 marker, each of these one or more marker(s) emitting a single signal otherwise known as the 'single detectable signal' (hereinafter the 'fifth signal'), different than the first four signals.

11. Use of a composition comprising at least:
- a κ chain marker and a λ chain marker emitting a different signal, and
- a marker selected from: a CD38 marker and a CD138 marker, said marker emitting a signal different than the first two signals, and
- one or more CD marker (s), preferentially at least two CD markers for normal plasma cells selected from CD19, CD27, CD45, CD81, CD5L, CD11a, CD16b, CD24, CD36, CD52, CD68, CD79a, CD80, CD82, CD84, CD99 and CD163 markers, preferentially CD19, CD27, CD45 and CD81 markers, more preferentially a CD19 marker and a CD27 marker, each of these one or more marker(s) emitting a single signal otherwise known as the 'single detectable signal' (hereinafter the 'fourth signal'), different than the first three signals, and
- one or more CD marker (s), preferentially at least two CD markers, more preferentially at least three CD markers for tumoral plasma cells selected from CD56, CD117, CD200, CD20, CD28, CD1D, CD2BP2, CD32c, CD47, CD59, CD109, CD229, CD300a and CD320 markers, preferentially CD56, CD117, CD200, CD20, and CD28 markers, more preferentially a CD56 marker, a CD117 marker, and a CD200 marker, each of these one or more marker(s) emitting a single signal otherwise known as the 'single detectable signal' (hereinafter the 'fifth signal'), different than the first four signals,
to carry out the process as claimed in one of claims 1 to 9.

12. A kit for carrying out the process as claimed in any one of claims 1 to 9, comprising at least:
- a κ chain marker and a λ chain marker, each emitting a different signal; and
- a CD38 marker or CD138 marker emitting a signal different than the first two signals; and
- one or more CD marker (s), preferentially at least two CD markers for normal plasma cells selected from CD19, CD27, CD45, CD81, CD5L, CD11a, CD16b, CD24, CD36, CD52, CD68, CD79a, CD80, CD82, CD84, CD99 and CD163 markers, preferentially CD19, CD27, CD45 and CD81 markers, more preferentially a CD19 marker and a CD27 marker, each of these one or more marker(s) emitting a single signal, otherwise known as the 'single detectable signal' (hereinafter the 'fourth signal'), different than the first three signals; and
- one or more CD marker (s), preferentially at least two CD markers, more preferentially at least three CD markers for tumoral plasma cells selected from CD56, CD117, CD200, CD20, CD28, CD1D, CD2BP2, CD32c, CD47, CD59, CD109, CD229, CD300a and CD320 markers, preferentially CD56, CD117, CD200, CD20, and CD28 markers, more preferentially a CD56 marker, a CD117 marker, and a CD200 marker, each of these one or more marker(s) emitting a single signal otherwise known as the 'single detectable signal' (hereinafter the 'fifth signal'), different than the first four signals.

13. The kit as claimed in claim 12, **characterized in that** it comprises at least one cell proliferation marker selected from:
- base analogs of BrdU type and an anti-BrdU antibody conjugated to APC or EdU and an azide coupled to a fluorochrome, preferentially Alexa Fluor® 647; and/or
- markers for proteins of the active cell cycle, for example an anti-Ki67 antibody conjugated to a fluorochrome, preferentially Alexa Fluor 647®; and/or
- deoxyribonucleic acid (DNA) markers, such as DAPI, PI, Hoechst dye (33342 or 33258),
each of said cell proliferation marker(s) emitting a signal different than the signals emitted by the other markers present in said kit, in particular different than the first five signals.
